# EUROPEAN PATENT SPECIFICATION

(11) **EP 1 641 813 B1**
(45) Date of publication and mention of the grant of the patent: **09.11.2011**
(21) Application number: 04809479.1
(22) Date of filing: 06.07.2004
(51) Int. Cl.: C07K 1/107, C07K 14/55

(54) **METHODS FOR OBTAINING MOLECULES WITH REDUCED IMMUNOGENICITY**
VERFAHREN ZUR HERSTELLUNG VON MOLEKÜLEN MIT VERMINDERTER IMMUNOGENITÄT
PROCEDES D'OBTENTION DE MOLECULES PRESENTANT UNE IMMUNOGENICITE REDUITE

(30) Priority: 03.07.2003 US 484880 P
(43) Date of publication of application: 05.04.2006
(73) Proprietor: Avatar Biotechnologies, Inc., Newark, NJ 07102 (US)
(72) Inventor: MARSHALL, Christopher, P., Brooklyn, NY 11217 (US)
(74) Representative: Lawrence, John
(86) International application number: PCT/US2004/021859
(87) International publication number: WO 2005/051975

(56) References cited:
- US-A1- 2002 061 549
- TAKANORI SO ET AL: "Depression of T-cell epitope generation by stabilizing hen lysozyme" JOURNAL OF BIOLOGICAL CHEMISTRY, vol. 272, no. 51, 19 December 1997 (1997-12-19), pages 32136-32140, XP002386260
- TADASHI UEDA ET AL.: "An intramolecular cross-linkage of lysozyme. Formation of cross-links between lysine-1 abd histidine-15 with Bis (bromoacetamide) derivatives by a two-stage reaction procedure and properties of the resulting derivatives" BIOCHEMISTRY, vol. 24, 22 October 1985 (1985-10-22), pages 6316-6322, XP002386261
- VIVEROS M ET AL: 'Characterization of a Novel Human Immunodeficiency Virus Type 1 Neutralizable Epitope within the Immunodominant Region of gp41.' VIROLOGY. vol. 270, no. 1, 25 April 2000, pages 135 - 145, XP004436053

## Description

This application claims priority of U.S. Serial No. 60/484,880, filed July 3, 2003.

This patent disclosure contains material that is subject to copyright protection. The copyright owner has no objection to the facsimile reproduction as it appears in the U.S. Patent and Trademark Office patent file or records, but otherwise reserves any and all copyright rights.

**Field Of The Invention**

The present invention relates to methods of engineering polypeptides, proteins and protein complexes to mask immunogenic epitopes (pharmaceutical, therapeutic, and industrial), and to polypeptides, proteins and protein complexes so engineered.

**Background of the Invention**

Due to the results of the human genome project and extensive biomedical research, the development period and time to market of protein-based therapeutic and diagnostic products administer to patients, for example, by injection, inhalation, or orally, are considered significantly shorter than that of chemically derived products. Furthermore, due to their high molecular specificity, biologically developed protein-based therapeutics are perceived to be more effective - and to have fewer side effects - than their chemical small molecule counterparts. Therefore, in recent years the pharmaceutical and biotechnology industries have focused significant attention on, and allocated significant resources toward, the development of protein-based therapeutics.

For example, the potential utility of antibodies, both as therapeutics and as diagnostic agents, has been recognized for many years. A significant number of technologies and methods of developing and engineering antibody-based products have emerged over the last decade or more, and immunoglobulin-base products now constitute one of largest and fastest growing segments of the pharmaceutical industry.
Takanori S. et al. ((1997) J. Biol. Chem. 272, 51, 32136-32140) discloses reduction of T-cell epitope generation and antigenicity by cross-linking and stabilization of the protein and reducing proteolysis by preventing endopeptidase access to cleavage sites.

### Summary Of The Invention

The invention provides for a method for reducing the immunogenicity of a peptide, protein or protein complex as defined in claim 1.

In one embodiment, the peptide, protein, or protein complex comprises a therapeutic product, a diagnostic product, an enzyme, a hormone, a receptor, a growth factor, an antibody or a fragment thereof.

In one embodiment, the cross-link comprises a homo-cross-link, a hetero-cross- link, a bifunctional cross-link, a photoreactive cross-link, a cross-link between non-classical amino acids incorporated into the peptide, or an oxidative cross-link.

The present invention provides a method for engineering/modifying polypeptides, proteins, and protein complexes to reduce and/or prevent undesirable immune responses. Polypeptides, proteins, and protein complexes are modified in such a way that the proteolytic fragments of these molecules that result from antigen processing are not displayed in the groove of the MHC, and/or T-helper cells specific for these peptides are not activated, or are activated to a lesser extent. Polypeptides, proteins, and/or protein complexes are engineered/modified by application of the instant invention in such a way that they retain or gain their desired activities and/or specificities, but are rendered incompatible with the structural requirements for specific biological processes in APC's that lead to T cell activation.

The specific biological processes in APC's that are inhibited or altered by application of the instant invention, and that alter MHC restricted antigen presentation and T-cell activation are the following:
- HLA-DM mediated peptide loading;
- Exopeptidase mediated proteolytic degradation of "dangling" sequences, i.e. sequences extending beyond either side of the groove of the MHC ("retrofitting", see above);
- HLA-DM mediated peptide editing;

Polypeptides, proteins, and protein complexes engineered by the method of the invention are crosslinked
- the peptides resulting from antigen processing are modified to the effect that they are rendered incompatible with the structural requirements for peptide loading and/or binding in the groove of the MHC;
- the peptides resulting from antigen processing are modified to the effect that they bind in the groove of the MHC with lower affinity, and are thus HLA-DM sensitive and subject to editing;
- the peptides resulting from antigen processing are modified to the effect that they are rendered incompatible with the structural requirements for exopeptidase-mediated "retrofitting", and are thus HLA-DM sensitive;

"HLA-DM sensitive" peptides are eliminated from the repertoire of displayed peptides (see above). Where peptide display is inhibited or prevented, peptide specific T-cell activation, and thus specific humoral immune responses are also inhibited or prevented. In cases where T cell signaling is incompletely inhibited, activated T-cells mediate the organism's acquisition of peptide specific tolerance.

### Brief Description Of The Figures

The present invention is now illustrated in connection with the accompanying drawings. The invention may be better understood by reference to one or more of these drawings in combination with the detailed description of the specific embodiments presented herein.

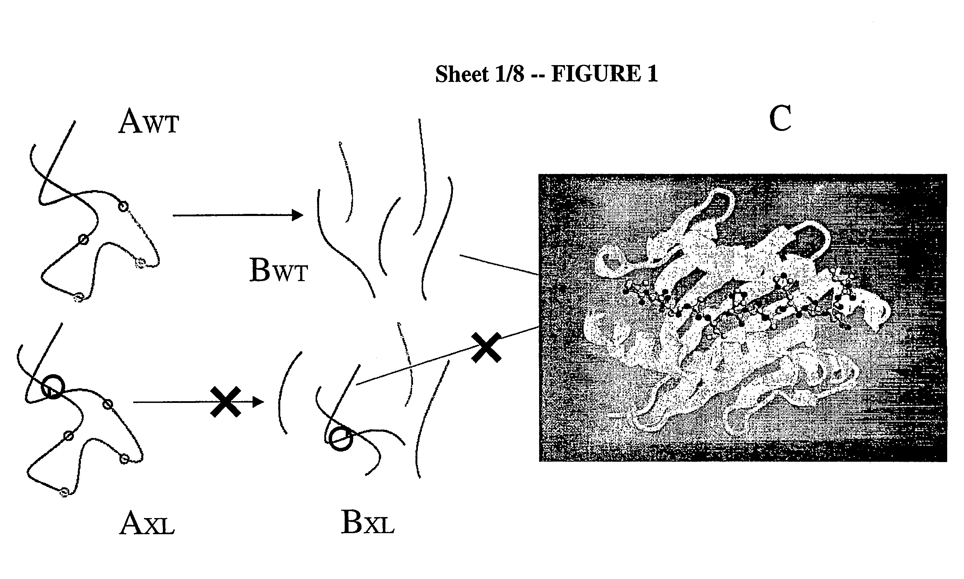

**Figure 1****.** Schematic/simplified representation of the biochemical pathway leading to MHCII presentation of an immunogenic peptide and the inventors' approach to blocking T-cell activation. A_{WT} represents the structure of an unmodified ("wild type") protein before antigen processing (partial proteolytic degradation); A_{XL} represents the structure of the protein after crosslinking; an immunogenic epitope is labelled "img.-epi". B_{WT} represents the set of peptides resulting from antigen processing of the unmodified protein, and the immunogenic peptide is labelled "img-pep"; B_{DT} represents the set of the set of peptides resulting from antigen processing of the cross-linked protein. C represents the peptide-loaded MHC. The immunogenic epitope of A is crosslinked and labelled "non-img-epi". D. D_{DT} represents the set of peptides resulting from antigen processing of the crosslinked protein, and the immunogenic peptide of B is crosslinked and labelled "non-img-pep". The arrows from A to B represent the proteolytic pathways; the arrows from B to C represent the pathways that lead to the formation of a stable peptide-MHC (pMHC) complex, including peptide loading, HLA-DM mediated editing, and exoproteolytic "trimming" of dangling sequences. An X crossing an arrow represents that this pathway is potentially impaired or blocked by application of the instant invention.

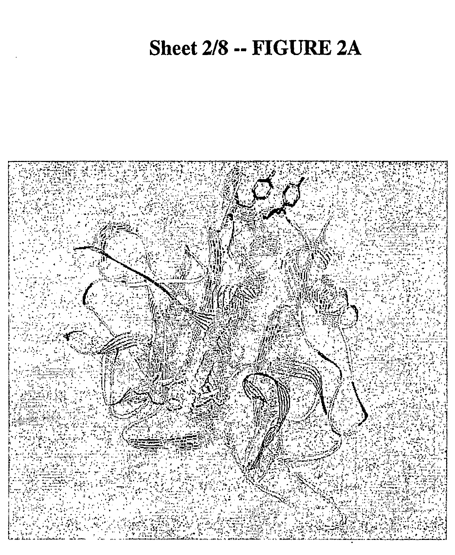

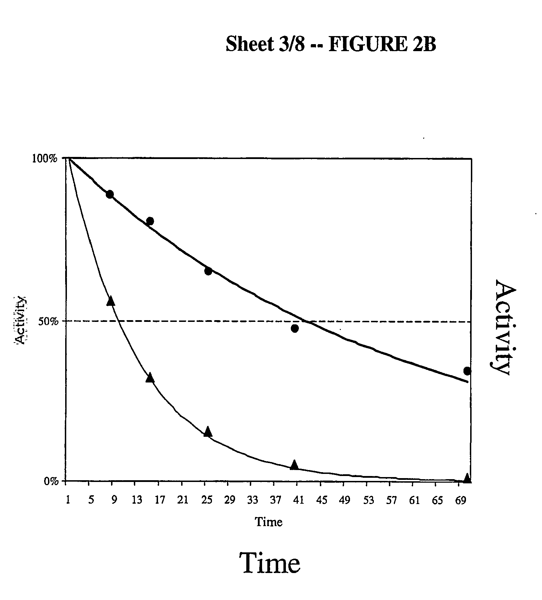

**Figures 2A-2B** - Reduced Auto-proteolytic Degradation of a Cross-linked, Promiscuous Protease. Figure 2A: Structural representation of Subtilisin E with K237Y point mutation to tyrosine prior to oxidative cross-linking. The ring structures near the top of the diagram are the tyrosine residues targeted for cross-linking (Y21, K237Y), and those near the bottom are are the residues of the active site. Figure 2B: Time-course heat inactivation experiments (colorometric assay) with DT crosslinked (DT) and wildtype (WT) Subtilisin E at 55°C. These results show that the protein structure is stabilized by a targeted (dityrosine) cross-link, and auto-proteolytic degradation is impaired leading to prolonged activity in time-course experiments.

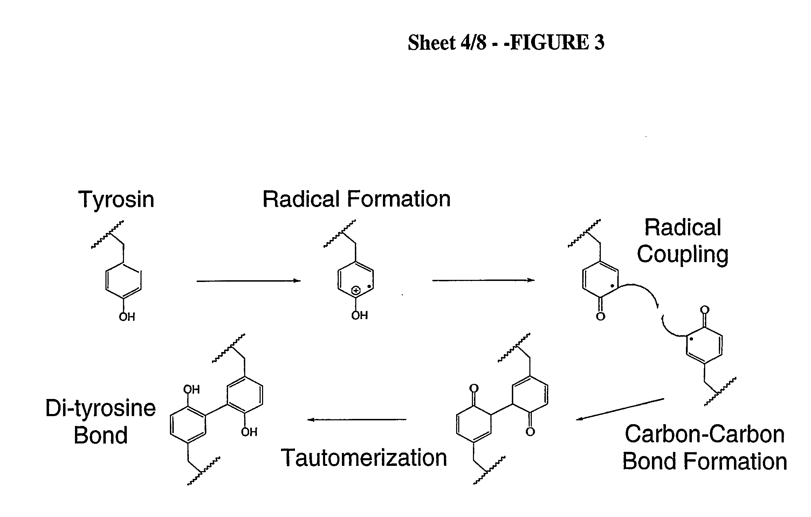

**Figure 3** - Schematic Representation of the putative chemical reactions and intermediates that lead to the formation of dityrosine bonds under oxidative conditions.

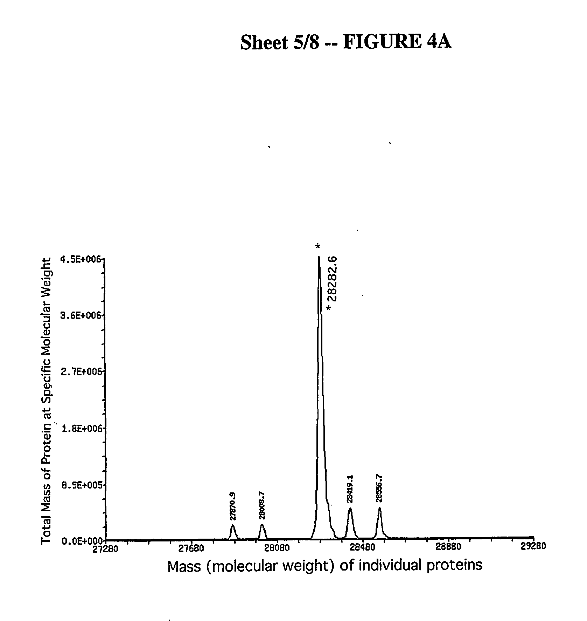

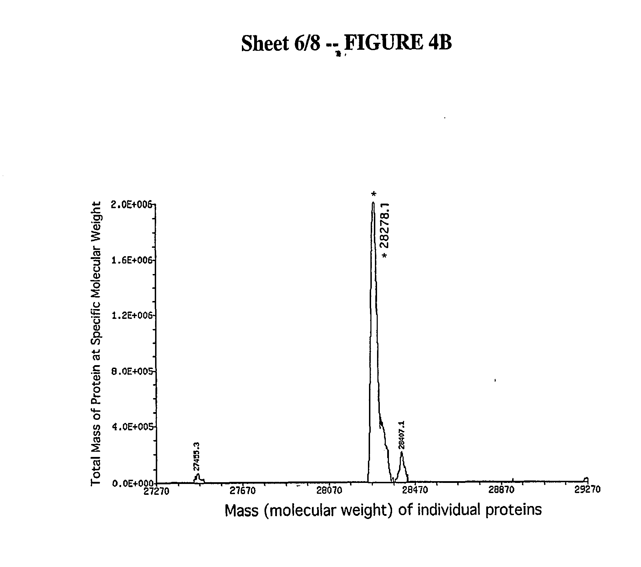

**Figures 4A-4B** - Diagram of Mass-spectrometric analysis of K237Y subtilisin E protein before and after dityrosine cross-linking. Figure 4A: Mass of the main peak before cross-linking: 28,282 Da. **B**: mass of the main peak after cross-linking: 28,278 Da. The predicted weight loss is 2 Da corresponding to the loss of two hydrogen atoms (see figure 3). Hardly any additional peak are present in Figure 4A or 4B. The accuracy of this assay is approximately +/- .01%. These results demonstrate that the protein remains intact upon exposure to cross-linking conditions.

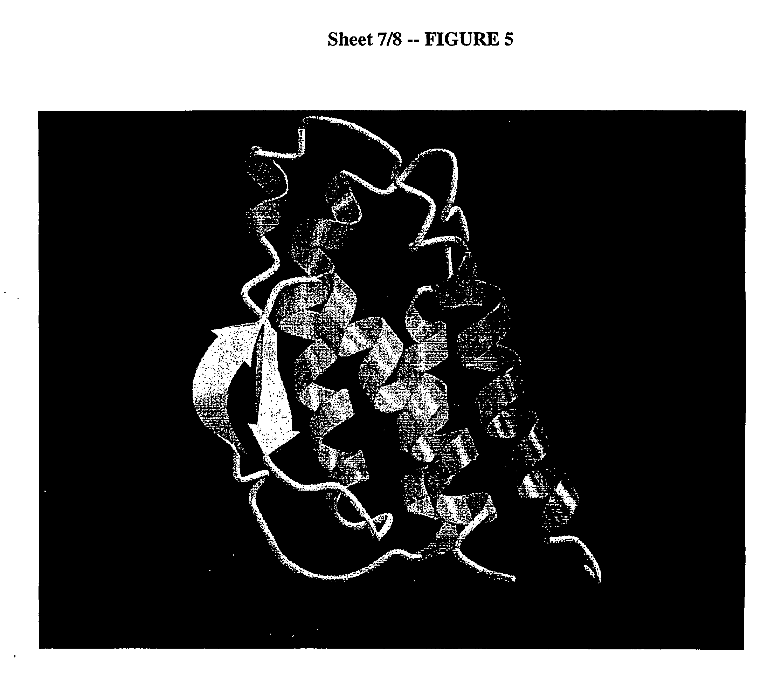

**Figure 5** - Structural representation of human IL-2. IL-2 is a member of the Short-chain 4 Alpha-helical Bundles with belong to the set of cytokines with four or five helix bundles (with alpha-spiral secondary structures).

### Detailed Description of the Invention

Polypeptides, proteins, and protein complexes are used as ingredients in wide variety of products, including, for example foods and detergents, in the production, manufacturing, and fabrication of wide variety of consumer products, such as, for example, textiles, paper, vitamins, food ingredients, and cosmetics, and in many industrial processes, such as, for example in fine chemical, pharmaceutical, and many other manufacturing processes.

**HUMORAL IMMUNE RESPONSES** - In order for the human immune system to mount responses to soluble protein-antigens, such as, for example, protein-based therapeutics ("biologics"), B-cells produce and secrete antibodies (immunoglobulin molecules) that bind to, sequester, and inactivate the antigen (humoral immune response). B cell activation requires activation of both of two sets of biochemical signaling pathways: (i) the signaling pathway triggered by binding of the B-cell receptor to its ligand, and (ii) the signaling pathway(s) triggered by antigen-specific CD4+ T-Helper cells, that, in turn, must also be activated (Villadangos JA et al., 1999. Immunol. Rev. 172:109-120). In the absence of T-cell activation, humoral immune responses to proteins are not triggered (see below).

Activation of B cells leads to clonal expansion and to secretion of antibodies specific to the antigen. The B-cell receptors bind to the antigen directly, and are the membrane bound variants of the specific immunoglobulin molecules the B cells will produce and secrete upon activation. On the other hand, T-cell activation, requires the collaboration of antigen presenting cells (APC's) that present to T-cells processed antigen fragments that are displayed in the groove of the major histocompatibility complex (MHC). T-cells are activated through recognition of MHC displayed antigen fragments through T-cell receptors (TCR's) specific to each T cell. By comparison to B-cell receptors (antibodies), that bind to molecules of almost any kind, T-cell receptors bind the more conserved structures of the surface of the peptide-MHC complex (pMHC), and the structural diversity of the TCR/pMHC is quite limited. Therefore, preventing T-cell activation is an effective method of inhibiting undesired immune responses.

Upon activation, T-Helper cells expand clonally, activate effector cells (B-cells, see above), and yield memory cells that allow the immune system to respond quickly and effectively to re-exposure to the same antigen. Proteins that enter APC's through the endocytic route, i.e. proteins not derived from the nucleoplasm or cytoplasm, are mostly displayed by MHC class II (MHC II). MHC II display of processed protein fragments involves uptake of the protein by receptor-mediated encocytosis, phagocytosis, macropinocytosis, and/or autophagy (Watts C, 1997. Annu. Rev. Immunol. 15: 821-850, Seglen PO & Bohley P., 1992. Experientia 48: 158-172, Villadangos JA et al., 1999. Immunol. Rev. 172:109-120), proteolyic digestion in the endocytic vesicle (Nakagawa TY & Rudensky AY, 1999. Immunol. Rev. 172:121-129; Villadangos JA et al., 1999. Immunol. Rev. 172:109-120), loading of the resultant peptide fragments into the groove of the MHC II, and transport of the peptide-loaded MHC II (pMHC II) to the plasma membrane (Alfonso C et al., 1999. Immunol. Rev. 172:255-266; Kropshofer H et al., 1999. Immunol. Rev. 172:267-278).

**ANTIGEN PROCESSEING** - In APCs, antigens presented by MHC class II molecules are taken up in endocytic vesicles, that progressively acidify, and in which the protein is degraded by proteases with acidic pH optima, such as the endopeptidases Cathepsin S and L, and the exopeptidases Cathepsins A, B and H (Nakagawa TY & Rudensky AY, 1999. Immunol. Rev. 172:121-129; Villadangos JA et al., 1999. Immunol. Rev. 172:109-120). Prior to proteolytic digestion, disulfide bonds are dissolved by thiol reductases, such as the Gamma Interferon inducible Lysosomal Thiol Reductase (GILT), which is expressed constitutively in APCs, localized in the MHC class II-containing lysosomal compartments, and has a low pH activity optimum (Phan UT, et al., 2000. J. Biol. Chem. 275 (34): 25907-14). Though it may not yet be as clearly established, it is thought that N-linked glycosylation is not removed in the endocytic and lysosomal compartments, and that N- and O-linked sugars are presented in the groove of the MHC class II molecules (Gad M et al., 2003. Eur. J. Immunol 33(6): 1624-32; Vlad AM et al., 2002, J. Exp. Med. 196 (11): 1435-46; Rudd PM et al., 2001. Science 291: 2370-76).

MHC LOADING AND PEPTIDE EDITING - In peptide loading compartments ("PLCs"); early and late endosomal, and lysosomal compartments, peptides are loaded into the groove of the MHC II molecules by either the "biosynthetic" route, or the "exchange" route. In the biosynthetic route, peptides are loaded in an HLA-DM mediated process in late endosomal and lysosomal compartments by which the invariant chain (Ii) derived CLIP peptide is displaced; peptide loading in the exchange route occurs in early endosomal compartments, and is also HLA-DM mediated (Alfonso C et al., 1999. Immunol. Rev. 172:255-266; Kropshofer H et al., 1999. Immunol. Rev. 172:267-278). Peptide editing, a process whereby certain antigens are removed again from the MHCII groove, is HLA-DM and HLA-DO mediated. Peptides that are edited are primarily thought to bind the groove of the MHC with low affinity, or to have extended sequences beyond the groove of the MHC (that "dangle"), particularly with extended sequences at the N-terminus. The affinity of the pMHC complex is determined by: (a) peptide anchor positions, particularly the primary anchor, which are hydrophobic amino acid side chains at specific positions in the sequence of the loaded peptide; (b) spacing of the peptide anchors and their steric fit with the corresponding binding sites in the groove of the MHC; and (c) presence or absence of destabilizing residues (often proline and glycine) in the amino acid positions in between the peptide anchors (Kropshofer H et al., 1999. Immunol. Rev. 172:267-278).

Peptides that are edited by the HLA-DO and HLA-DM system are referred to as DM- sensitive, and peptides that are not edited, and that are then presented to T-cells for recognition and activation are referred to as DM-resistant once the pMHC reaches the surface of the APC (Kropshofer H et al., 1999. Immunol. Rev. 172:267-278).

**T-Cell Activation** - T-cell activation follows binding of the pMHC to its specific TCR, assembly of the extra-and intracellular signaling complexes, and activation of the intracellular signaling pathways. For all of these events to occur, it is necessary for the TCR- pMHC complex to assemble in such a way that the other members of the signaling complex can also associate with the complex, and lead to biochemical, transcriptional and cellular activation.

The TCR is generally thought to be diagonally oriented across the surface of the pMHC, whereby the CDR3, which are centrally disposed, interacts with the central, up facing amino acid side chains of the peptide bound in the groove of the MHC. The CDRs 1 and 2 of the TCR contact more conserved elements of the MHC, which may include conserved helical structures/residues. The alpha chain of the TCR lies over, and contacts the N-terminus of the MHC bound peptide, and the beta chain lies over the its C-terminus. Upon binding significant conformational changes can occur on both the TCR and the pMHC, which may modify the kind of signaling that occurs, and which may also be directly involved in activation of the biochemical signaling pathways (Garcia KC, 1999. Immunol. Rev. 172:73-85).

**Detrimental Humoral Immune Responses**

**Toxicity** - In cases where significant quantities of a protein to which antibodies are generated, such as, for example protein-based therapeutics, blood substitutes, cytokines, cytokine traps, antibodies, antibody fragments, fusion proteins, and biocatalysts/industrial enzymes, antibody-antigen complexes can have toxic effects, for example with regard to kidney function, where such complexes can "clog" the glomeruli, and prevent the kidneys from filtering and secreting toxic substances from the body (Mire-Sluis A et al., 2003. Dev Bio (Basel) 112: 153-63), as, for example, in the cases of interferon alpha, and interferon beta (see Table 1).

**Anaphylactic Shock** - In cases where an immunogenic peptide, polypeptide, protein, or protein complex is taken up by an organism, whereby immunogenic peptides reach the bloodstream and cause immune responses, for example by inhalation, a powerful immune response to, for example a bacterial protein, such as the protease subtilisin, can cause anaphylactic shock, and in some cases, death (Malkiel S & Hargis BJ, 1971. J Allergy Clin Immunol. 48(4): 220-3).

**Reduced Efficacy** - The development of therapeutic proteins is regarded as one of the most promising areas of drug development, for reasons such as the achievable therapeutic specificity due to their structural complexity, and their projected short times to market. Immune responses to such potentially therapeutic proteins, however, such as, but not limited to, blood substitutes, cytokines, cytokine traps, antibodies, antibody fragments, fusion proteins, and derivatives, such as, for example radio-labeled antibodies, and generally protein-based products of any kind, lead to the production of antibodies specific to the these molecules that bind to, inactivate, and/or sequester them, and reduce and sometimes even negate their therapeutic potential (Mire-Sluis A et al., 2003. Dev Bio (Basel) 112: 153-63).

In cases where the immune response is directed at epitopes that are conserved or similar between two or several proteins with therapeutic potential, an immune response to one protein may produce antibodies that will also bind to, inactivate and/or sequester the other protein(s), and render patients untreatable by a whole range of otherwise potentially potent medicines and treatment methods.

Table 1: Reported Clinical Consequences of Immune Responses to Select Biopharmaceuticals

| Biopharmaceutical | Primary Clinical Consequence |
|---|---|
| Growth hormone | Decrease in activity |
| Insulin | Resistance |
| EPO | Red cell aplasia |
| Factor VIII | Decrease in activity |
| IFN-α | Decrease in activity |
| IFN-β | Decrease in activity |
| Anti-CD3 Mab | Increase CD3 |
| PEG-MDGF | Thrombocytopenia |

From " Assessing Interferon Immunogenicity;" a presentation by Ron Bordens

**Crossreactivity and Autoimmunity** - Immune responses to non-self proteins can lead to the production of antibodies that cross react with proteins that are produced by the body itself, recognition, inactivation, and/or sequestration of which can have negative effects on the bodies ability to perform important functions. For example, immune reactions to erythropoietin caused several cases of (irreversible) pure red cell aplasia, a life threatening disorder (Berry JD, et al., 2003. Hybrid Hybridomics 22(1): 23-31), and immune reactions to PEG MDGF lead to thrombocytopenia (see Table 1). Furthermore, the induction of cross- reactive immune responses can lead to autoimmune disorders.

**Immune Evasion Strategies** - Several mechanisms are known that allow non-self, i.e. foreign, proteins to evade immune recognition. These mechanisms have both naturally evolved and are the result of protein engineering.

**In Nature** - Posttranslational modifications of proteins that are not removed during antigen processing, such as glycosylation, can render modified peptides that result from such a protein's processing non compatible with MHC presentation, and thus such modified peptides do not, or cannot, illicit an immune responses (Surman S et al., 2001. PROC NATL ACAD SCI USA 98(8): 4587-92; Rudd PM et al., 2001. Science 291: 2370-76). This is one of the mechanisms that human physiology exploits toward distinguishing between self and non-self polypeptides, proteins and protein complexes, such as the MHC classes I and II themselves, antibodies, and many other important intra- and extracellular proteins. There are also many mechanisms known in nature by which pathogenic viruses and intracellular bacteria and parasites evade or subvert immune responses, many of which involve modulation and/or prevention of MHC class I and class II display. In many of these systems co-evolution of the host and/or its immune system and the pathogen and/or its immune evasion tactic is evident (Brodsky FM et al., 1999. Immunol. Rev. 168:199-215; Bennett EM et al., 1999. J Immunol. 162(9): 5049-52).

For example, the E19 protein of Adenovirus prevents MHC class I (MHC I) antigen presentation by inhibiting MHC I expression through two mechanisms: (i) direct binding to the MHC I heavy chain effecting cytoplasmic retention (i.e. prevention of MHC I heavy chain trafficking to the peptide loading compartments and to the plasma membrane), and hence inhibition of peptide loading and MHC display; (ii) direct binding to the TAP protein involved in peptide translocation of peptides resulting from cytoplasmic antigen processing to the peptide loading compartments, and hence inhibition of peptide loading.

Another example is the Epstein Barr Virus, which has evolved to avoid antigen processing and display by evading antigen processing. Proc Natl Acad Sci USA 94, 12616-21). There exists an extensive literature on this issue (see, as examples, Fruh K et al., 1997, Ploegh HL, 1998; Tortorella D et al, 2000; Yewdell JW & Bennink JR, 1999; Hengel H & Kaszinowski UH, 1997; Peters VB & Sperber KE, 1999; Maksymowych WP & Kane KP, 2000; Yewdell JW & Hill AB, 2002).

**Protein Engineering** - Given the often very negative effects of immune responses to protein-based therapeutics (biologics) and other protein-based products, there is significant value in developing technologies that allow proteins to evade the immune system. Several technologies are currently being applied to products on the market and in development. Such technologies include, as examples, PEGylation and epitope identification and subsequent amino acid sequence modification, for example, by applying molecular biological methods, such as the introduction of (point) mutation(s).

PEGylation of proteins involves the attachment of polyethylene glycol (PEG) moieties on the surface of proteins that render a protein invisible to the immune system by avoiding receptor recognition. The chemical structures of PEG moieties render the surfaces of PEGylated proteins structurally mistakable for water. Furthermore, PEGylation has been demonstrated to interfere with antigen processing (proteolytic degradation, see above; So T et al., 1996. Immunology Letters 49 (1-2): 91-97). PEGylation does not, however, necessarily interfere with peptide presentation, as PEGylated peptides have been demonstrated to form stable complexes with the groove of MHC molecules (Bouvier M & Wiley DC, 1996. Proc Natl Acad Sci U S A. 93(10): 4583-8). Though this technology has been, and remains, very successful, there are limits to its applicability: for example, immunogenic epitopes on proteins that are in the active site or relevant protein-protein interaction sites mostly cannot be engineered to avoid immune responses.

Epitope identification and subsequent amino acid sequence modification is also a widely applied approach in product development. The identification of immunogenic epitopes is achieved either physically or computationally. Physical methods of epitope identification include, for example, mass spectrometry and tissue culture/cellular techniques. Computational approaches make use of information obtained on antigen processing, loading and display, structural and/or proteomic data toward identifying non-self peptides that could/should result from antigen processing, and that are likely to have good binding characteristics in the groove of the MHC. One or more mutations are then introduced into the gene(s) directing the expression of the desired protein(s), that maintain its functionality and simultaneously render the identified epitope less or non-immunogenic.

**Protein Modifications** - Several examples of polypeptide, protein, and protein complex modifications that are not reversible/removable during antigen processing, and that could be engineered into the structure of a peptide, protein, or protein complex to interfere with antigen processing and peptide loading are known, such as, for example, glycosylation and PEGylation, as described above.

**Protein Crosslinking** - A vast literature, and a wide variety of methods of crosslinking proteins intro- and intermolecularly are also known with varying lengths of spacer arms, and with and without fluorescent and functional groups for purification. These methods include the use of heterobifunctional crosslinkers (e.g. succinimidyl acetylthioacetate (SATA), trans-4-(maleimidylmethyl) cyclohexane-1-carboxylate (SMCC), and succinimidyl 3-(2-pyridyldithio)propionate (SPDP)), homobifunctional crosslinkers (e.g. succinimidyl 3-(2-pyridyldithio)propionate), photoreactive crosslinkers (e.g. 4-azido-2,3,5,6-tetrafluorobenzoic acid, STP ester, sodium salt (ATFB, STP ester), 4-azido-2,3,5,6-tetrafluorobenzoic acid, succinimidyl ester (ATFB, SE), 4-azido-2,3,5,6-tetrafluorobenzyl amine, hydrochloride, benzophenone-4-isothiocyanate, benzophenone-4-maleimide, 4-benzoylbenzoic acid, succinimidyl ester, N-((2-pyridyldithio)ethyl)-4- azidosalicylamide (PEAS; AET), thiol reactive crosslinkers (e.g. maleimides and iodoacetamides), amine reactive crosslinkers (e.g. glutaraldyde, bis(imido esters), bis(succinimidyl esters), diisocyanates and diacid chlorides).. Because thiol groups are highly reactive and relatively rare in most proteins by comparison to amine groups, thiol-reactive crosslinking is generally preferred. In cases where thiol groups are missing at the appropriate sites in the structures of polypeptides, proteins, and protein complexes, they can be introduced using one of several thiolation methods. For examples, Succinimidyl trans-4-(maleimidylmethyl)cyclohexane-1-carboxylate can be used to introduce thiol-reactive groups at amine sites.

Furthermore, several oxidative crosslinks are known, such as dityrosine bonds that are highly stable, and irreversible under the conditions of antigen processing.

**Dityrosine Bonds** - Dityrosine ("DT") bonds occur in nature as a result of protein oxidation. This chemical process either is a part of a physiological system, in which DT bond formation evolved to exploit the characteristics of the bond (see below), or is the result of oxidative stress and/or aging, which in many cases leads to the protein's denaturation and inactivation (Kanwar & Balasubramanian, 2000, Biochemistry 39, 14976). DT bond formation is thought to be irreversible under any and all physiological conditions, and hence serum DT concentrations are used as an effective diagnostic marker for oxidative stress and aging (Galeazzi L et al., 1999, Amyloid 6:7-13; Pennathur S et al., 1999. J. Biol. Chem. 274: 34621-28; Ziouzenkova O et al., 1999. J. Biol. Chem. 274: 18916-18924; Leeuwenbergh C et al., 1997. J. Biol. Chem. 272: 3520-3526; Abdelrahim M et al., 1997. J. Chromatogr., B: Biomed. Sci. 696: 175-82; Kato Y et al., 1998. FEBS Lett. 439: 231-234; van der Vliet A et al., 1998. J. Biol. Chem. 273: 31860-31866; Garcia-Castineiras S et al., 1987. Exp. Eye Res. 26: 464-476; Wells-Knecht MC et al., 1993. J. Biol. Chem. 268: 12348-52; Onorato JM et al., 1998. Ann. N.Y. Acad. Sci. 854: 277-90; Leeuwenbergh C et al., 1999. Free Radical Biol. Med. 27, 186-92). Several proteins are described that contain DT bonds in their functional state, and their function is also thought to be dependent on the presence of the DT bond. However, such proteins are not terribly abundant in biological systems, which is presumably also a function of the irreversible nature of the crosslink. Examples of proteins that are thought to require DT bonds for function include the chicken elastin in the aortic wall (LaBella F et al., 1967. Biochem. Biophys. Res. Commun. 26, 748), resilin, a protein present in the cuticle of insects (Andersen, SO, 1966. Acta Physiol. Scand. 66 Suppl., 263), bamboo cell wall proteins (Totsune et al., 1993. Biochem. Biophys. Res. Commun. 194: 1025), and human periodontal ligament collagen (Tenovuo, J. and Paunio, K., 1979. Arch. Oral Biol. 24, 591). Furthermore, DT bonds provide the molecular basis of the structure and function of gluten in foods, such as bread (Tilley KA, 2001. J. Agric. Food Chem 49, 2627)

Thus, DT bonds are not likely to be toxic or immunogenic per se, and particularly important for the purposes of the instant invention, highly stable in endocytosis vesicles and lysosomes of APCs during antigen processing.

DT bonds are zero-length crosslinks, i.e. no atoms are added, and only two hydrogen protons are lost, and thus targeted DT crosslinking is thought to be an advantageous method of protein engineering from the standpoint of preserving the activity, specificity, and structural integrity of a polypeptide, protein, or protein complex. Furthermore, it has been shown that (i) only tyrosyl side chains form crosslinks under the oxidative conditions that lead to DT bond formation, and (ii) that only tyrosyl side chains in close structural proximity form DT bonds. Thus targeting DT bonds to specific locations within the structure of a polypeptide, protein, or protein complex is possible, patented (Marshall CP et al., US patent application No. 09/837,235), and demonstrated (Brown KC et al., 1998. Biochemistry 37: 4397-4406.).

**Polypeptides And Proteins Containing Non-Clasical Amino Acid Sidechains** - As methods of protein synthesis, structural prediction algorithms, and/or evolutionary techniques and selection methods based on functional properties progress and improve, the inclusion of non-classical amino acids, described below, becomes more and more an economically viable method of producing protein-based products. Non-classical amino acids may contain reactive groups, and thus engineering cross-linked amino acid side chains into protein-based products may become attractive.

**Polypeptides, Proteins And Protein Complexes Suitable For Application Of The Invention** - Polypeptides, proteins, and protein complexes that can be engineered by the methods described herein comprise polypeptides consisting of at least 6 amino acids, 10 amino acids, 20 amino acids, 50 amino acids, 100 amino acids, 200 amino acids, 500 amino acids, 1000 amino acids, 2000 amino acids, or of at least 5000 amino acids, and complexes that consist of two or more polypeptides and that remain functionally active upon application of the instant invention. Nucleic acids encoding the foregoing polypeptides are also provided. The term "functionally active" material, as used herein, refers to that material displaying one or more functional activities or functionalities associated with one or more of the polypeptides of the complex. Such activities or functionalities may be the polypeptide complexes' original, natural or wild type activities or functionalities, or they may be designed and/or engineered. Such design and/or engineering may be achieved, for example, either by deleting amino acids, or adding amino acids to, parts of one, any, both, several, or all of the polypeptides, by fusing polypeptides of different proteins or protein complexes, by adding or deleting post-translational modifications, by adding chemical modifications or appendixes, or by introducing any other mutations by any methods known in the art to this end as set forth in detail below.

In specific embodiments, the invention provides fragments of an engineered polypeptide or polypeptide complex consisting of at least 6 amino acids, 10 amino acids, 20 amino acids, 50 amino acids, 100 amino acids, 200 amino acids, 500 amino acids, 1000 amino acids, 2000 amino acids, or of at least 5000 amino acids.

**Polypeptide Derivatives And Analogs** - Derivatives or analogs of proteins include those molecules comprising regions that are substantially homologous to a protein or fragment thereof (e.g., in various embodiments, at least 40% or 50% or 60% or 70% or 80% or 90% or 95% identity over an amino acid or nucleic acid sequence of identical size or when compared to an aligned sequence in which the alignment is done, for example, by a computer homology program known in the art) or whose encoding nucleic acid is capable of hybridizing to a coding gene sequence, under high stringency, moderate stringency, or low stringency conditions.

Further, one or more amino acid residues within the sequence can be substituted by another amino acid of a similar polarity that acts as a functional equivalent, resulting in a silent alteration. Substitutions for an amino acid within the sequence may be selected from other members of the class to which the amino acid belongs. For example, the nonpolar (hydrophobic) amino acids include alanine, leucine, isoleucine, valine, proline, phenylalanine, tryptophane and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Such substitutions are generally understood to be conservative substitutions.

The derivatives and analogs of the polypeptides of the complex to be engineered by application of the instant invention can be produced by various methods known in the art. The manipulations that result in their production can occur at the gene or protein level. For example, a cloned gene sequence can be modified by any of numerous strategies known in the art.

Chimeric polypeptides can be made comprising one or several of the polypeptides of a complex to be engineered by the instant invention, or fragment, derivative, analog thereof (preferably consisting of at least a domain of a protein complex to be engineered, or at least 6, and preferably at least 10 amino acids of the protein) joined at its amino- or carboxy- terminus via a peptide bond to an amino acid sequence of a different protein. Such a chimeric polypeptide can be produced by any known method, including: recombinant expression of a nucleic acid encoding the polypeptide (comprising a polypeptide coding sequence joined in-frame to a coding sequence for a different polypeptide); ligating the appropriate nucleic acid sequences encoding the desired amino acid sequences to each other in the proper coding frame, and expressing the chimeric product; and protein synthetic techniques, for example, by use of a peptide synthesizer.

**Manipulations Of A Protein Sequence At The Protein Level** -Included within the scope of the invention are polypeptides or polypeptide fragments, which are differentially modified during or after translation or synthesis, for example, by crosslinking, glycosylation, acetylation, phosphorylation, amidation, derivatization by known protecting/blocking groups or proteolytic cleavage. Any of numerous chemical modifications may be carried out by known techniques, including specific chemical cleavage by cyanogen bromide, trypsin, chymotrypsin, papain, V8 protease, NaBH₄, acetylation, formylation, oxidation, reduction or metabolic synthesis in the presence of tunicamycin. In addition, polypeptides or polypeptide fragments that can be stabilized using the methods of the instant invention can be chemically synthesized. For example, a peptide corresponding to a portion of a protein can be synthesized by use of a peptide synthesizer. Furthermore, if desired, non-classical amino acids or chemical amino acid analogs can be introduced as substitutions and/or additions into the sequence of one, any, both, several or all of the polypeptides of a complex.

Non-classical amino acids include the D-isomers of the common amino acids, fluoro-amino acids, designer amino acids such as β-methyl amino acids, Cγ-methyl amino acids, Nγ-methyl amino acids, and amino acid analogs in general. Examples of non-classical amino acids include: α-aminocaprylic acid, Acpa; (S)-2-aminoethyl-L-cysteine/HCl, Aecys; aminophenylacetate, Afa; 6-amino hexanoic acid, Ahx; γ-amino isobutyric acid and α-aminoisobytyric acid, Aiba; alloisoleucine, Aile; L-allylglycine, Alg; 2-amino butyric acid, 4-aminobutyric acid, and α-aminobutyric acid, Aba; p-aminophenylalanine, Aphe; b-alanine, Bal; p-bromophenylalaine, Brphe; cyclohexylalanine, Cha; citrulline, Cit; β-chloroalanine, Clala; cycloleucine, Cle; p-cholorphenylalanine, Clphe; cysteic acid, Cya; 2,4-diaminobutyric acid, Dab; 3-amino propionic acid and 2,3-diaminopropionic acid, Dap; 3,4-dehydroproline, Dhp; 3,4-dihydroxylphenylalanine, Dhphe; p-flurophenylalanine, Fphe; D-glucoseaminic acid, Gaa; homoarginine, Hag; δ-hydroxylysine/HCl, Hlys; DL-β-hydroxynorvaline, Hnvl; homoglutamine, Hog; homophenylalanine, Hoph; homoserine, Hos; hydroxyproline, Hpr; p-iodophenylalanine, Iphe; isoserine, Ise; α-methylleucine, Mle; DL-methionine-S-methylsulfoniumchloide, Msmet; 3-(1-naphthyl) alanine, 1Nala; 3-(2-naphthyl) alanine, 2Nala; norleucine, Nle; N-methylalanine, Nmala; Norvaline, Nva; O-benzylserine, Obser; O-benzyltyrosine, Obtyr; O-ethyltyrosine, Oetyr; O-methylserine, Omser; O-methylthreonine, Omthr; O-methyltyrosine, Omtyr; Ornithine, Orn; phenylglycine; penicillamine, Pen; pyroglutamic acid, Pga; pipecolic acid, Pip; sarcosine, Sar; t-butylglycine; t-butylalanine; 3,3,3-trifluroalanine, Tfa; 6-hydroxydopa, Thphe; L-vinylglycine, Vig; (-)-(2R)-2-amino-3-(2-aminoethylsulfonyl) propanoic acid dihydroxochloride, Aaspa; (2S)-2-amino-9-hydroxy-4,7-dioxanonanoic acid, Ahdna; (2S)-2-amino-6-hydroxy-4-oxahexanoic acid, Ahoha; (-)-(2R)-2-amino-3-(2-hydroxyethylsulfonyl) propanoic acid, Ahsopa; (-)-(2R)-2-amino-3-(2-hydroxyethylsulfanyl) propanoic acid, Ahspa; (2S)-2-amino-12-hydroxy-4,7,10-trioxadodecanoic acid, Ahtda; (2S)-2,9-diamino-4,7-dioxanonanoic acid, Dadna; (2S)-2,12-diamino-4,7,10-trioxadodecanoic acid, Datda; (S)-5,5-difluoronorleucine, Dfnl; (S)-4,4-difluoronorvaline, Dfnv; (3R)-1-1-dioxo-[1,4]thiaziane-3-carboxylic acid, Dtca; (S)-4,4,5,5,6,6,6-heptafluoronorleucine, Hfnl; (S)-5,5,6,6,6-pentafluoronorleucine, Pfnl; (S)-4,4,5,5,5-pentafluoronorvaline, Pfnv; and (3R)-1,4-thiazinane-3-carboxylic acid, Tca. Furthermore, the amino acid can be D (dextrorotary) or L (levorotary). For a review of classical and non-classical amino acids, see Sandberg *et al*. (Sandberg M. et al. J. Med. Chem. 41(14): pp. 2481-91,1998).

**Molecular Biological Methods** - Nucleic acids encoding one or more polypeptides engineered by the methodology of instant invention are provided. The polypeptides of the complex can be made by expressing the DNA of the complex can be made by expressing the DNA sequences that encode them *in vitro* or *in vivo* by any known method in the art. Nucleic acids encoding the complex to be stabilized by the methodology of the instant invention can be made by altering the nucleic acid sequence encoding the polypeptide or polypeptides by substitutions, additions (e.g., insertions) or deletions that provide for functionally active molecules. The sequences can be cleaved at appropriate sites with restriction endonuclease(s), followed by further enzymatic modification if desired, isolated, and ligated *in vivo* or *in vitro*. Additionally, a nucleic acid sequence can be mutated *in vitro* or *in vivo*, to create and/or destroy translation, initiation, and/or termination sequences, or to create variations in coding regions and/or to form new, or destroy preexisting, restriction endonuclease sites to facilitate further *in vitro* modification. Due to the degeneracy of nucleotide coding sequences, many different nucleic acid sequences which encode substantially the same amino acid sequence as a complex to be engineered may be used in the practice of the present invention. These can include nucleotide sequences comprising all or portions of a domain which is altered by the substitution of different codons that encode the same amino acid, or a functionally equivalent amino acid residue within the sequence, thus producing a "silent" (functionally or phenotypically irrelevant) change. Any technique for mutagenesis known in the art can be used, including chemical, mutagenesis, *in vitro* site-directed mutagenesis, using, for example, the QuikChange Site-Directed Mutagenesis Kit (Stratagene).

**Application Of The Immune Evasion Technology**

**Protein modifications** - Polypeptides, proteins and or protein complexes are modified chemically in such a way that the modifications are irreversible *in vivo* and/or under the conditions of antigen processing, and such that epitopes of unmodified proteins identified as immunogenic (see above) are no longer displayed by MHC II molecules, and thus no longer immunogenic. Such irreversible modifications are formed by crosslinks, such as, the formation crosslinks, such as, , crosslinks resulting from the use of homo- and hetero-bifunctional crosslinking agents that react with amine and/or thiol groups, photoreactive crosslink reagents, any crosslinks that may form between non-classical amino acids incorporated into the structure of a polypeptide, protein, or protein complexes, as described above, and any oxidative crosslinks, such as, dityrosine bonds, as described above.

These modifications are placed/targeted to such sites in the structure of polypeptides, proteins, and protein complexes where the desired specificity and/or activity of the molecule/complex is maintained or the desired specificity and/or activity is in part a result of the modification. Alternatively, polypeptides, proteins, and/or protein complexes with the desired modifications may be isolated from a mixture of modified and unmodified proteins with and without the desired modifications based on chemical, physical, and/or functional characteristics. Such characteristics may include, as examples, molecular weight, molecular volume, any and all chromatographic properties, mobility in any all forms of electrophoresis, any and all biochemical characteristics, fluorescence and any and all other biophysical characteristics, solubility in aqueous solutions, (organic) solvents, and/or hybrid solutions in the presence or absence of other molecules in solution (e.g. ions) at different concentrations, affinity to mono- and/or polyclonal antibodies, affinity to receptors, other proteins, DNA, RNA, lipids, other bio- and non-bio-organic molecules and complexes, inorganic molecules and complexes, ions, any and all structural characteristics, enzymatic, immunological, tissue culture, diagnostic, pharmaceutical, and any other activity or activities, and many other characteristics.

**Targeted biological processes in APC's that are altered, inhibited, or prevented -** The instant invention provides a method of inhibiting and/or altering biological processes in APC's that lead to MHC restricted antigen presentation on the surface of APCs. The specific biological processes that are affected by application of the instant invention are the following:
- HLA-DM mediated peptide loading;
- Exopeptidase mediated proteolytic degradation of "dangling" sequences, i.e. sequences extending beyond either side of the groove of the MHC ("retrofitting", see above);
- HLA-DM mediated peptide editing;

**Products to which invention can be applied to -** The instant invention is meaningfully applied to any and all protein-based products that (i) are immunogenic or have immunogenic epitopes, and (ii) suffer negative consequences with regard to their potential as therapeutics, diagnostics, food or detergent additives, industrial enzymes, and in the market place from said immunogenic properties (see above). Application is facilitated when the technology is applied to products with discrete immunogenic epitopes that can be, or have been, identified using any methods known to one skilled in the art, as described above. Proteins and/or protein derivatives to which the instant invention can be applied may fall into any category of protein-based products, including therapeutic and diagnostic proteins, proteins included in products, such as, for example to, foods and detergents, proteins used in the manufacturing processes of products, such as, for example to, textiles and paper, and others, and protein with relevance in industrial manufacturing, such as biocatalysts, and any derivatives of any such proteins.

Therapeutic Products - Therapeutic protein-base products to which the instant invention can be applied may, for example, act as cytokines that trigger/induce biochemical signaling cascades, and cellular and physiological responses, by binding to, and activating, receptors on the surface of targeted cells. Examples of cytokines include any of the interferons, any of the interleukins, members of the NFG/TGF family (e.g. NGF, TGF, BDNF, NT-3, NT-4/5, NT-6, TRAIL, OPG, and FasL), any of the colony stimulating factors (e.g. M-CSF, G-CSF, and GM-CSF), any of the FGF family, any members of the insulin family, EGF and related cytokines, VEGF, and PDGF and related cytokines. On the other hand, therapeutic, protein-based products to which the instant invention can be applied may act as cytokine traps, which are protein constructs that include the extracellular domains of cytokine receptors, and that bind to, sequester, and inactivate endogenous cytokines. Examples of cytokine traps include the IL-1, IL-4/13, and the VEGF Traps by Regeneron Inc.

Therapeutic protein-based products to which the instant invention can be applied may also be immunoglobulin-based products. Such products may be complete immunoglobulin molecule complexes, or may consist only of fragments thereof. Such fragments may contain the Fc fragment with and without complement binding domains, Fab and/or F(ab)₂ fragments, Fv fragments stabilized by any method known to one skilled in the art, including single chain Fv fragments ("scFv"), disulfide Fv fragments ("dsFv"), and/or any combination of methods applied together.

Other products to which the instant invention can be applied may be polypeptides, proteins and protein complexes with catalytic activity - enzymes - blood substitution products, such as, for example, hemoglobin and Factor IIX, extra- and intracellular matrix proteins, peptide hormones, and any other polypeptides, proteins, protein complexes that are naturally occurring and/or were otherwise evolved, selected and/or designed.

In addition, such products may be fusion proteins consisting in part, as examples, of a complete, or one or more domains or segments of, immunoglobulin, albumin, or any extracellular matrix protein molecule and/or complex. Such products may, by way of illustration, be targeted to particular molecules, complexes, cells, tissues, and/or organs, by fusing a protein domain or segment with a desired activity, such as an enzymatic or cytokinetic activity ("effector domain") with a domain that targets the fusion protein or protein complex, such as, for example, the variable, antigen binding domain (Fv fragment) of an immunoglobulin molecule or an extracellular matrix binding domain ("targeting domain"). In Table 1, a list of approved therapeutic products that may benefit from application of the instant invention.

**Diagnostic Products** - Protein-based products that are administered to patients orally, by injection, by inhalation, or by any other acceptable methods known in the art, for diagnostic purposes also constitute a subset of the polypeptides, proteins, and protein complexes, to which the instant invention can be applied. These products may, for example, target a signal or tracer to a specific tissue, organ, tumor, or any other body mass and can be used to visualize and diagnose abnormalities, conditions, and diseases. Such products can also elicit immune responses with detrimental effects, as described above. These products can be based on any of the above categories of polypeptides, proteins, or protein complexes.

**Other Products** - Polypeptides, proteins and protein complexes used as ingredients in food products, detergents, and any other consumer products, or that in any other way can be ingested, inhaled, or come in contact with the skin or any other organs of a human body, and that can or may cause immune responses with detrimental effects, as described above, constitute a subset of the polypeptides, proteins, and protein complexes, to which the instant invention can be applied.

**Table 2: Six classes of immunogenic biopharmaceuticals**

| **Recombinant DNA** | **Recombinant DNA** | **Recombinant DNA** |
|---|---|---|
| **products homologous to** | **products** | **products** |
| **native proteins** | **Hybrid molecules** | **Sequence variants** |
| Growth hormone | GM- CSF/ IL3 | Interferon alpha consensus |
| Interferon alpha 2 | TNFR55/ IgG1 | Methionyl HGH |
| Interferon beta | TNFR70/ IgG1 | |
| GM- CSF | Denileukin- diftitox | |
| EPO | | |
| Insulin | | |
| IL-2 | | |
| GnRH | | |
| Desoxyribonuclease | | |
| HCNTF | | |

| **Recombinant DNA** | **Natural products of** | **Products of non- human** |
|---|---|---|
| **products modified** | **human origin** | **origin** |
| **chemically** | | |
| Pegylated interferon alpha- | 2 Growth hormone | Insulin |
| Pegylated MDGF | Factor VIII | Streptokinase |
| | Glucocerebrosidase | Staphylokinase |
| | HCG | ADA |
| | | Calcitonin |
| | | Thrichosantin |

| | | |
|---|---|---|
| From "Immunogenicity of biopharmaceutical, the European perspective," a presentation by Huub Schelecken. | | |

**Obtaining Polypeptides, Proteins And Protein Complexes To Be Engineered** - Any method known to one skilled in the art may be used to obtain a polypeptide or polypeptide complex to be engineered according to the methods of the invention.

**Purification Of Polypeptides** - A polypeptide or polypeptides of a complex to be engineered using the methods of the instant invention may be obtained, for example, by any protein purification method known in the art. Such methods include, chromatography (*e*.*g*. ion exchange, affinity, and/or sizing column chromatography), ammonium sulfate precipitation, centrifugation, differential solubility, or by any other standard technique for the purification of proteins. The polypeptides may be purified from any source that produces one, any, both, several or all of the polypeptides of a complex of the desired complex to be stabilized. For example, polypeptides may be purified from sources including, prokaryotic, eukaryotic, mono-cellular, multi-cellular, animal, plant, fungus, vertebrate, mammalian, human, porcine, bovine, feline, equine, canine, avian, tissue culture cells, and any other natural, modified, engineered, or any otherwise not naturally occurring source. The degree of purity may vary, but in various embodiments, the purified protein is greater than 50%, 75%, 85%, 95%, 99%, or 99.9% of the total mg protein. Thus, a crude cell lysate would not comprise a purified protein.

Where it is necessary to introduce mutations into a purified protein or protein complex, the protein(s) can be micro-sequenced to determine a partial amino acid sequence. The partial amino acid sequence can then be used together with library screening and recombinant nucleic acid methods well known in the art to isolate the clones necessary to introduce desired mutations.

EXPRESSION OF DNA ENCODING A POLYPEPTIDE

Source of DNA

Any prokaryotic or eukaryotic cell can serve as the nucleic acid source for molecular cloning. A nucleic acid sequence encoding a protein or domain to be engineered may be isolated from sources including prokaryotic, eukaryotic, mono-cellular, multi-cellular, animal, plant, fungus, vertebrate, mammalian, human, porcine, bovine, feline, equine, canine, avian, etc.

The DNA may be obtained by standard procedures known in the art from cloned DNA (e.g., a DNA "library"), by chemical synthesis, by cDNA cloning, by the cloning of genomic DNA, or fragments thereof, purified from the desired cell (see e.g., Sambrook *et al*., 1985. Glover (ed.). MRL Press, Ltd., Oxford, U.K.; vol. I, II). The DNA may also be obtained by reverse transcribing cellular RNA, prepared by any of the methods known in the art, such as random- or poly A-primed reverse transcription. Such DNA may be amplified using any of the methods known in the art, including PCR and 5' RACE techniques (Weis J.H. et al.,, 1992. Trends Genet. 8(8): 263-4; Frohman MA, , 1994. PCR Methods Appl. 4(1): S40-58).

Whatever the source, the gene should be molecularly cloned into a suitable vector for propagation of the gene. Additionally, the DNA may be cleaved at specific sites using various restriction enzymes, DNAse may be used in the presence of manganese, or the DNA can be physically sheared, as for example, by sonication. The linear DNA fragments can then be separated according to size by standard techniques, such as agarose and polyacrylamide gel electrophoresis and column chromatography.

Cloning

Once the DNA fragments are generated, identification of the specific DNA fragment containing the desired gene may be accomplished in a number of ways. For example, clones can be isolated by using PCR techniques that may either use two oligonucleotides specific for the desired sequence, or a single oligonucleotide specific for the desired sequence, using, for example, the 5' RACE system (Cale JM et al., 1998. Methods Mol. Biol. 105: 351-71; Frohman MA, 1994. PCR Methods Appl. 4(1): S40-58). The oligonucleotides may or may not contain degenerate nucleotide residues. Alternatively, if a portion of a gene or its specific RNA or a fragment thereof is available and can be purified and labeled, the generated DNA fragments may be screened by nucleic acid hybridization to the labeled probe (e.g. Benton and Davis, 1977. Science 196(4286): 180-2). Those DNA fragments with substantial homology to the probe will hybridize. It is also possible to identify the appropriate fragment by restriction enzyme digestion(s) and comparison of fragment sizes with those expected according to a known restriction map if such is available. Further selection can be carried out on the basis of the properties of the gene.

The presence of the desired gene may also be detected by assays based on the physical, chemical, or immunological properties of its expressed product. For example, cDNA clones, or DNA clones which hybrid-select the proper mRNAs, can be selected and expressed to produce a protein that has, for example, similar or identical electrophoretic migration, isoelectric focusing behavior, proteolytic digestion maps, hormonal or other biological activity, binding activity, or antigenic properties as known for a protein.

Using an antibody to a known protein, other proteins may be identified by binding of the labeled antibody to expressed putative proteins, for example, in an ELISA (enzyme-linked immunosorbent assay)-type procedure. Further, using a binding protein specific to a known protein, other proteins may be identified by binding to such a protein either in vitro or a suitable cell system, such as the yeast-two-hybrid system (see e.g. Clemmons DR, 1993. Mol. Reprod. Dev. 35: 368-74; Loddick SA, 1998 et al. Proc. Natl. Acad. Sci., U.S.A. 95:1894-98).

A gene can also be identified by mRNA selection using nucleic acid hybridization followed by *in vitro* translation. In this procedure, fragments are used to isolate complementary mRNAs by hybridization. Such DNA fragments may represent available, purified DNA of another species (e.g., Drosophila, mouse, human). Immunoprecipitation analysis or functional assays (e.g. aggregation ability *in vitro*, binding to receptor, etc.) of the *in vitro* translation products of the isolated products of the isolated mRNAs identifies the mRNA and, therefore, the complementary DNA fragments that contain the desired sequences.

In addition, specific mRNAs may be selected by adsorption of polysomes isolated from cells to immobilized antibodies specifically directed against protein. A radiolabeled cDNA can be synthesized using the selected mRNA (from the adsorbed polysomes) as a template. The radiolabeled mRNA or cDNA may then be used as a probe to identify the DNA fragments from among other genomic DNA fragments.

Alternatives to isolating the genomic DNA include, chemically synthesizing the gene sequence itself from a known sequence or making cDNA to the mRNA, which encodes the protein. For example, RNA for cDNA cloning of the gene can be isolated from cells that express the gene.

Vectors

The identified and isolated gene can then be inserted into an appropriate cloning or expression vector. A large number of vector-host systems known in the art may be used. Possible vectors include plasmids or modified viruses, but the vector system must be compatible with the host cell used. Such vectors include bacteriophages such as lambda derivatives, or plasmids such as PBR322 or pUC plasmid derivatives or the Bluescript vector (Stratagene).

The insertion into a cloning vector can, for example, be accomplished by ligating the DNA fragment into a cloning vector that has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. Furthermore, the gene and/or the vector may be amplified using PCR techniques and oligonucleotides specific for the termini of the gene and/or the vector that contain additional nucleotides that provide the desired complementary cohesive termini. In alternative methods, the cleaved vector and a gene may be modified by homopolymeric tailing (Cale JM et al., 1998. Methods Mol. Biol. 105: 351-71). Recombinant molecules can be introduced into host cells via transformation, transfection, infection, electroporation, etc., so that many copies of the gene sequence are generated.

Preparation of DNA

In specific embodiments, transformation of host cells with recombinant DNA molecules that incorporate an isolated gene, cDNA, or synthesized DNA sequence enables generation of multiple copies of the gene. Thus, the gene may be obtained in large quantities by growing transformants, isolating the recombinant DNA molecules from the transformants and, when necessary, retrieving the inserted gene from the isolated recombinant DNA.

The sequences provided by the instant invention include those nucleotide sequences encoding substantially the same amino acid sequences as found in native proteins, and those encoded amino acid sequences with functionally equivalent amino acids, as well as those encoding other derivatives or analogs, as described below for derivatives and analogs.

Structure of Genes and Proteins

The amino acid sequence of a protein can be derived by deduction from the DNA sequence, or alternatively, by direct sequencing of the protein, for example, with an automated amino acid sequencer.

A protein sequence can be further characterized by a hydrophilicity analysis (Hopp TP & Woods KR, 1981. Proc. Natl. Acad. Sci., U.S.A. 78: 3824). A hydrophilicity profile can be used to identify the hydrophobic and hydrophilic regions of the protein and the corresponding regions of the gene sequence, which encode such regions.

Secondary, structural analysis (Chou PY & Fasman GD, 1974. Biochemistry 13(2): 222-45) can also be done, to identify regions of a protein that assume specific secondary structures. Manipulation, translation, and secondary structure prediction, open reading frame prediction and plotting, as well as determination of sequence homologies, can also be accomplished using computer software programs available in the art. Other methods of structural analysis include X-ray crystallography, nuclear magnetic resonance spectroscopy and computer modeling.

**Suitable Residues For Engineering**

**Targeting Strategies**

Any method known to one skilled in the art may be used to identify T-cell epitopes of a polypeptide, protein, or protein complex that are immunogenic, whereby such potential T cell epitopes are defined as any peptide within the protein sequence with the ability to bind to the groove of MHC molecules for presentation to the immune system. MHC binding of such potential epitopes is measured by any computational or physical method known to one skilled in the art. T cell epitopes are recognized by T cell receptors and, given the presence of T-cells bearing T-cell receptors specific for such an epitope in the context of MHC presentation, trigger activation of such T cells. Such methods may be based on the methods described in Estell DA, 2003. (U. S. Patent No. 6,642,011, "Human protease and use of such protease for pharmaceutical applications and for reducing the allergenicity of non-human proteins"), Estell DA & Harding FA, 2001 (U.S. Patent No. 6,218,165, "Mutant proteins having lower allergenic response in humans and methods for constructing, identifying and producing such proteins"), PCT International Publication No. WO-A-9852976, computational algorithms, such as, for example, identification of MHC binding peptides using computational threading methods (Altuvia et al., 1995. J. Mol. Biol. 249 244-250), any physical methods, such as, for example, mass spectrometry of displayed peptides upon exposure of protein to APCs, chromatographic and/or electorphoretic analysis of labeled and unlabeled polypeptides, proteins, and protein complexes degraded by proteases known to be involved in proteolytic degradation of proteins in the endocytic/lysosomal compartments of APCs, chromatographic and/or electorphoretic analysis of displayed peptides upon exposure of APCs to radio- or otherwise-labeled protein, T-cell activation assays, such as, T cell proliferation assays (Adorini L et al., 1988. J. Exp. Med. 168: 2091; So T. et al., 1996. Immunol. Let. 49: 91-97) and IL-2 or other cytokine production by proliferative response assays of CTLL-2 cells (Gillis S et al., 1978. J. Immunol. 120: 2027; So T. et al., 1996. Immunol. Let. 49: 91-97) and recombinant expression of peptides, peptide synthesis, or enzymatic protein degradation in combination with T cell activation assays and/or methods of tissue culture.

Residues contained in peptides resulting from antigen processing that contain such immunogenic epitopes are modified by molecular biological, and/or chemical methods that include directed or undirected crosslinking, Specifically, polypeptides, proteins and protein complexes are modified to the effect that:
- the peptides resulting from antigen processing are modified to the effect that they are rendered incompatible with the structural requirements for peptide loading and/or binding in the groove of the MHC;
- the peptides resulting from antigen processing are modified to the effect that they bind in the groove of the MHC with lower affinity, and are thus HLA-DM sensitive and subject to editing;
- the peptides resulting from antigen processing are modified to the effect that they are rendered incompatible with the structural requirements for exopeptidase-mediated "retrofitting", and are thus HLA-DM sensitive;

Polypeptides, proteins, and protein complexes may be modified such that they are not, or such that they are incompletely, processed upon crosslinking, and they, or their intermediate degradation products gain or retain intramolecular structures and/or folds during antigen processing Such structures and/or folds may interfere with peptide loading and/or high affinity binding in the groove of the MHC. Alternatively, due to the same structures and/or folds and therefore also as a result of incomplete processing, the resulting peptides may not be "retrofitted" and would then retain "dangling" sequences, which render peptides HLA-DM sensitive and subject to editing, as described above.

Polypeptides, proteins, and protein complexes may also be engineered in such a way that modified peptides that result from their antigen processing attain intramolecular structures that interfere with the structural requirements for loading and/or high affinity binding in the groove of the MHC. In a particular embodiment, application of the instant invention provides crosslinked polypeptides, proteins, or protein complexes that in antigen processing yield non-linear peptides, whereby the crosslinked residue would otherwise be displayed within the groove of the MHC. Thereby, the proteolytic fragments of these molecules that result from antigen processing either
- contain one or more peptide loops; or
- are crosslinked dipeptides with four peptide arms extending from the crosslinked pair of amino acids

Peptides that are loaded and presented in the MHC are normally linear. In either of the above cases, the modified peptides resulting from antigen processing are either incompatible with the structural requirements for peptide loading and/or binding in the groove of the MHC, or their affinity for the groove of the MHC is rendered lower, and they thereby become HLA-DM sensitive.

Polypeptides, proteins, and protein complexes may furthermore be modified in such a way that antigen processing, and peptide loading into the groove of the MHC are not affected, but that "retrofitting" of modified peptides in the groove of the MHC is inhibited, and dangling sequences are not removed. In a particular embodiment of the instant invention, peptides resulting from antigen processing are crosslinked, and the resultant structures described above are incompatible with the structural requirements for exopeptidase-mediated retrofitting. Dangling sequences render the peptides HLA-DM sensitive and subject to editing, as described above.

"HLA-DM sensitive" peptides are eliminated from the repertoire of displayed peptides by the HLA-DM and HLA-DO mediated editing processes described. Where peptide display is inhibited or prevented, peptide specific T-cell activation, and thus specific humoral immune responses are also inhibited or prevented.

In cases where T cell signaling is incompletely inhibited, activated T-cells mediate the organism's acquisition of peptide specific tolerance.

**Maintenance of Proteins' Activity and/or Specificity** - Any method known to one skilled in the art may be used to identify, select, and/or target residues for modifications that ensure that a protein with reduced immunogenicity either maintains its activity and/or specificity, or assumes a desired activity or specificity upon modification. Alternatively, modified polypeptides, proteins, and/or protein complexes with reduced immunogenicity and retained activity and/or specificity may be isolated from a mixture of modified and unmodified proteins with and without the desired modifications based on chemical, physical, and/or functional characteristics, as described above.

**DNA Vector Constructs** - The nucleotide sequence coding for the polypeptide, or for one, any, both, several or all of the polypeptides of a complex, or functionally active analogs or fragments or other derivatives thereof, can be inserted into an appropriate expansion or expression vectors, i.e., a vector which contains the necessary elements for the transcription alone, or transcription and translation, of the inserted protein-coding sequence(s). The native genes and/or their flanking sequences can also supply the necessary transcriptional and/or translational signals.

Expression of a nucleic acid sequence encoding a polypeptide or peptide fragment may be regulated by a second nucleic acid sequence so that the polypeptide is expressed in a host transformed with the recombinant DNA molecule. For example, expression of a polypeptide may be controlled by any promoter/enhancer element known in the art.

Promoters which may be used to control gene expression include, as examples, the SV40 early promoter region, the promoter contained in the 3' long terminal repeat of Rous sarcoma, the herpes thymidine kinase promoter, the regulatory sequences of the metallothionein gene; prokaryotic expression vectors such as the β-lactamase promoter, or the lac promoter; plant expression vectors comprising the nopaline synthetase promoter or the cauliflower mosaic virus 35S RNA promoter, and the promoter of the photosynthetic enzyme ribulose biphosphate carboxylase; promoter elements from yeast or other fungi such as the Gal 4 promoter, the alcohol dehydrogenase promoter, phosphoglycerol kinase promoter, alkaline phosphatase promoter, and the following animal transcriptional control regions, which exhibit tissue specificity and have been utilized in transgenic animals: elastase I gene control region which is active in pancreatic acinar cells (Swift et al., 1984. Cell 38: 639-46); a gene control region which is active in pancreatic beta cells (Hanahan D, 1985. Nature 315: 115-22), an immunoglobulin gene control region which is active in lymphoid cells (Grosschedl R et al., 1984. Cell; 38: 647-58), mouse mammary tumor virus control region which is active in testicular, breast, lymphoid and mast cells (Leder A et al., 1986. Cell; 45: 485-95), albumin gene control region which is active in liver (Pinkert CA et al., 1987. Genes Dev. 1: 268-76), alpha-fetoprotein gene control region which is active in liver (Krumlauf R et al., 1985. Mol. Cell. Biol. 5: 1639-48); alpha 1-antitrypsin gene control region which is active in the liver (Kelsey GD et al., 1987. Genes Dev. 1: 161-71), betaglobin gene control region which is active in myeloid cells (Magram J et al., 1985 Nature 315: 338-40); myelin basic protein gene control region which is active in oligodendrocyte cells in the brain (Readhead C et al., 1987 Cell 48: 703-12); myosin light chain-2 gene control region which is active in skeletal muscle (Shani M, 1985. Nature 314: 283-86), and gonadotropic releasing hormone gene control region which is active in the hypothalamus (Mason AJ et al., 1986. Science 234: 1372-78).

In a specific embodiment, a vector is used that comprises a promoter operably linked to a gene nucleic acid, one or more origins of replication, and, optionally, one or more selectable markers (e.g., an antibiotic resistance gene). In bacteria, the expression system may comprise the lac-response system for selection of bacteria that contain the vector. Expression constructs can be made, for example, by subcloning a coding sequence into one the restriction sites of each or any of the pGEX vectors (Pharmacia, Smith DB & Johnson His, 1988. Gene 67: 31-40). This allows for the expression of the protein product.

Vectors containing gene inserts can be identified by three general approaches: (a) identification of specific one or several attributes of the DNA itself, such as, for example, fragment lengths yielded by restriction endonuclease treatment, direct sequencing, PCR, or nucleic acid hybridization; (b) presence or absence of "marker" gene functions; and, where the vector is an expression vector, (c) expression of inserted sequences. In the first approach, the presence of a gene inserted in a vector can be detected, for example, by sequencing, PCR or nucleic acid hybridization using probes comprising sequences that are homologous to an inserted gene. In the second approach, the recombinant vector/host system can be identified and selected based upon the presence or absence of certain "marker" gene functions (e.g., thymidine kinase activity, resistance to antibiotics, transformation phenotype, occlusion body formation in baculovirus, etc.) caused by the insertion of a gene in the vector. For example, if the gene is inserted within the marker gene sequence of the vector, recombinants containing the insert an identified by the absence of the marker gene function. In the third approach, recombinant expression vectors can be identified by assaying the product expressed by the recombinant expression vectors containing the inserted sequences. Such assays can be based, for example, on the physical or functional properties of the protein in *in vitro* assay systems, for example, binding with anti-protein antibody.

Once a particular recombinant DNA molecule is identified and isolated, several methods known in the art may be used to propagate it. Once a suitable host system and growth conditions are established, recombinant expression vectors can be propagated and prepared in quantity. Some of the expression vectors that can be used include human or animal viruses such as vaccinia virus or adenovirus; insect viruses such as baculovirus; yeast vectors; bacteriophage vectors (e.g., lambda phage), and plasmid and cosmid DNA vectors.

Once a recombinant vector that directs the expression of a desired sequence is identified, the gene product can be analyzed. This is achieved by assays based on the physical or functional properties of the product, including radioactive labeling of the product followed by analysis by gel electrophoresis, immunoassay, etc.

**Systems Of Gene Expression And Protein Purification**

A variety of host-vector systems may be utilized to express the protein-coding sequences. These include, as examples, mammalian cell systems infected with virus (e.g., vaccinia virus, adenovirus, etc.); insect cell systems infected with virus (e.g., baculovirus); microorganisms such as yeast containing yeast vectors, or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

In a specific embodiment, the gene may be expressed in bacteria that are protease deficient, and that have low constitutive levels and high induced levels of expression where an expression vector is used that is inducible, for example, by the addition of IPTG to the medium.

In yet another specific embodiment the polypeptide, or one, any, both, several or all of the polypeptides of a complex may be expressed with signal peptides, such as, for example, pelB bacterial signal peptide, that directs the protein to the bacterial periplasm (Lei et al., 1987. J. Bacterol. 169: 4379). Alternatively, protein may be allowed to form inclusion bodies, and subsequently be resolubilzed and refolded (Kim SH et al., 1997. Mol. Immunol 34: 891).

In yet another embodiment, a fragment of the polypeptide, or one, any, both, several or all of the polypeptides a complex comprising one or more domains of the protein is expressed. Any of the methods previously described for the insertion of DNA fragments into a vector may be used to construct expression vectors containing a chimeric gene consisting of appropriate transcriptional/translational control signals and the protein coding sequences.
These methods may include in vitro recombinant DNA and synthetic techniques and in vivo recombinants (genetic recombination).

In addition, a host cell strain may be chosen that modulates the expression of the inserted sequences, or modifies and processes the gene product in the specific fashion desired. Expression from certain promoters can be elevated in the presence of certain inducers; thus, expression of the genetically engineered polypeptides may be controlled. Furthermore, different host cells have characteristic and specific mechanisms for the translational and post-translational processing and modification (e.g. protein glycosylation, phosphorylation). Appropriate cell lines or host systems can be chosen to ensure the desired modification and processing of the foreign polypeptide(s) expressed. For example, expression in a bacterial system can be used to produce a non-glycosylated core protein product. Expression in yeast will produce a glycosylated product. Expression in mammalian cells can be used to ensure "native" glycosylation of a heterologous protein. Furthermore, different vector/host expression systems may effect processing reactions to different extents.

In other embodiments of the invention the polypeptide or one, any, both, several or all of the polypeptides a complex, and/or fragments thereof may be expressed as a fusion-, or chimeric, protein product (comprising the protein, fragment, analog, or derivative joined via a peptide bond to a heterologous protein sequence of a different protein). Such a chimeric product can be made by ligating the appropriate nucleic acid sequences encoding the desired amino acid sequences to each other by methods known in the art, in the proper coding frame, and expressing the chimeric product by methods commonly known in the art. Alternatively, such a chimeric product may be made by protein synthetic techniques, for example, by use of a peptide synthesizer.

The polypeptides of a complex may be expressed together in the same cells either on the same vector, driven by the same or independent transcriptional and/or translational signals, or on separate expression vectors, for example by cotransfection or cotransformation and selection, for example, may be based on both vectors' individual selection markers. Alternatively, one, any, both, several or all of the polypeptides a complex may be expressed separately; they may be expressed in the same expression system, or in different expression systems, and may be expressed individually or collectively as fragments, of the original polypeptide.

**Engineering Polypeptides, Proteins And Protein Complexes**

Once a protein to which the instant invention can be applied is prepared, expressed, and/or purified, as described above, a chemical modification can be applied in order to avoid presentation of an immunogenic peptide's presentation in the MHC and/or activation of T-helper cells specific to said immunogenic epitope. Any one, or a combination of any of the above mentioned targeting strategies and protein modification methods may be used. Alternatively, the modification may not be targeted, and proteins with the desired modifications, activities, and/or specificities may be isolated from a mixture of modified and unmodified proteins.

**Dt Bond Formation** - In one particular embodiment, dityrosine ("DT") bonds/crosslinks are targeted to specific residue pairs within the structure of a polypeptide, protein, or protein complex, as described above, where DT bonds will, or are predicted to form, due to, for example, their close proximity. This is either done with proteins in which at the targeted residue pair(s) tyrosyl sidechains are already present, and other tyrosyl side chains will not, or are not predicted to, form DT bonds because, for example, they are not in close enough proximity to each other. Otherwise polypeptides and proteins are engineered in such a way that at the targeted residue pair(s), tyrosyl sidechains are present, and at residues where undesirable DT crosslinks may form, at least one of the tyrosyl sidechains is replaced with a phenylalanine sidechain (Marshall CP et al., US patent application No. 09/837,235). This may be achieved, for example, by introducing point mutations to tyrosine in the gene(s) directing the expression of a polypeptide, protein, or proteins of a complex by any methods known to one skilled in the art. Alternatively, the polypeptides, proteins, and proteins of a complex may be synthesized, purified, or produced by any methods known to one skilled in the art (see above).

Proteins with tyrosyl sidechains at the targeted residue pair(s) are then subjected to reaction conditions that lead to the formation of DT bonds. Such conditions are, or become, oxidative reaction conditions, as the DT bond formation reaction is an oxidative crosslink. In the preferred embodiment, reaction conditions yield proteins that are otherwise not, or not detectably, modified. Such conditions are obtained by use of enzymes that catalyze the formation of H₂O₂, such as peroxidases. DT bond formation is monitored by spectrophotometry with an excitation wavelength of 320 nm, and fluorescence measured at a wavelength of 400 nm, while loss of tyrosyl fluorescence is monitored also monitored by standard procedures. When loss of tyrosyl florescence is no longer stoicometric with DT bond formation, the reaction is stopped by any methods known to one skilled in the art, such as, for example, the addition of a reducing agent and subsequent cooling (on ice) or freezing of the sample.

**Purification Of Engineered Polypeptides, Proteins And Protein Complexes**

The engineered polypeptide, protein, or protein complex may be isolated and purified from other proteins in the reaction, or any other undesirable side-products, by standard methods including chromatography (e.g., sizing column chromatography, glycerol gradients, affinity), centrifugation, or by any other standard technique for the purification of proteins. In specific embodiments it may be necessary to separate polypeptides that were not cross-linked, but that homo- or heterodimerize with other polypeptides due to high affinity binding. Separation may be achieved by any means known in the art, including, for example, addition of detergent and/or reducing agents.

Yield of functionally engineered polypeptides, proteins, and protein complexes can be determined by any means known in the art, for example, by comparing the amount of engineered material, purified as described above, with the starting material. Protein concentrations are determined by standard procedures, such as, for example, Bradford or Lowrie protein assays. The Bradford assay is compatible with reducing agents and denaturing agents (Bradford, M, 1976. Anal. Biochem. 72: 248), the Lowry assay is better compatibility with detergents and the reaction is more linear with respect to protein concentrations and read-out (Lowry, O J, 1951. Biol. Chem. 193: 265).

Assay Of Engineered Polypeptides, Proteins And Protein Complexes

**Immunogenicity** - The immunogenicity of a polypeptide, protein, or protein complex may be assayed by any method known to one skilled in the art. Most commonly, the presence (or absence), titres, affinities, avitidies, etc. of antibodies generated *in vivo* are tested by standard methods, such as, but not limited to, ELISA assays, by which the above parameters are tested on immunoglobulin present in the serum of an organism (or patient). Additional methods, such as generating T-cell hybridomas and measuring activation in the presence of APCs and antigen (Surman S et al., 2001 Proc. Natl. Acad. Sci. USA 98: 4587-92, below), examining labeled or unlabeled MHC presented peptides by chromatography, electorphoresis, and/or mass spectroscopy, T-cell activation assays, such as, but not limited to, T cell proliferation assays (Adorini L et al., 1988. J. Exp. Med. 168: 2091; So T. et al., 1996. Immunol. Let. 49: 91-97) and IL-2 production by proliferative response assays of CTLL-2 cells (Gillis S et al., 1978. J. Immunol. 120: 2027; So T. et al., 1996. Immunol. Let. 49: 91-97), and many others may be applied to determine more specific aspects of an immune response, or the lack thereof, such as, for example, the identity of the immunogenic T cell epitope of the antigen.

As specific examples, *in vitro* T cell assays may be carried out whereby the polypeptide, protein, or protein complex can be processed and presented in the groove of MHC molecules by appropriate antigen-presenting cells (APCs) to syngeneic T cells. T cell responses may be measured by simple proliferation measurements or by measuring release of specific cytokine by activated cells; APCs may be irradiated or otherwise treated to prevent proliferation to facilitate interpretation of the results of such assays. In order to determine the immunogenicity of an epitope in the context of different MHC allotypes, in vivo assays using syngeneic APCs and T-cells of a range of allotypes may be carried out to test for T cell epitopes in a range of individuals or patients.

Alternatively, transgenic animals expressing MHC molecules from human (or any other species of interest) maybe used to assay for T cell epitopes; in a preferred embodiment this assay is carried out in transgenic animals in which the endogenous MHC repertoire has been knocked out and, better yet, in which one or more other accessory molecules of the endogenous MHC/T cell receptor complex have also been replaced with human molecules (or molecules of any other species of interest), such as, for example, the CD4 molecule.

Furthermore, to detect anti-protein/antigen antibodies directly in vivo, for example in clinical and animal studies, ELISA assays, such as, for example solid phase indirect ELISA assays, may be used to detect binding of antibodies. In one specific embodiment, microtiter plates are incubated with the peptide, protein, or protein complex of interest at an appropriate concentration and in a suitable buffer. After washes with an appropriate washing solution, such as, for example PBS (pH 7.4), PBS containing 1% BSA and 0.05% Tween 20, or any other such solution as may be appropriate, serum samples are diluted, for example in PBS/BSA, and equal volumes of the samples are added in duplicate to the wells. The plates are incubated, and after additional washes, for example with PBS, anti-immunoglobulin antibodies coupled/conjugated to a reporter, such as a radioactive isotope or alkaline phosphatase, are added to each well at an appropriate concentration, and incubated. The wells are then washed again, and for example, in the case of use of alkaline phosphatase as a reporter, the enzyme reaction is carried our using a colorometric substrate, such as p-nitrophenyl phosphate in diethanolamine buffer (pH 9.8), absorbance of which can be read at 405 nm, for example, in an automatic ELISA reader (e.g. Multiskan PLUS; Labsystems).

As an additional example, to detect antibodies in the serum of patients and animals, immunoblotting can also be applied. In one specific embodiment, an appropriate amount of the peptide, protein, or protein complex of interest per samples/lane is run on gels (e.g. polyacrylamide), under reducing and/or nonreducing conditions, and the protein is transferred to a membrane, such as, for example, PVDF membranes; any other method to separate proteins by size can be used followed by transfer of the protein to a membrane. The membranes are blocked, for example, using a solution of 5% (w/v) milk' powder in PBS. The blots are then incubated with serum samples at varying dilutions in the blocking solution (before and after injection regimen) and control anti-antigen, so far as such samples are available. The blots will be washed four times with an appropriate washing solution, and further incubated with reporter-conjugated anti- immunoglobulin at a appropriate/specified dilutions for appropriate/specified periods of time under appropriate/specified conditions. The blots are washed again with an appropriate washing solution, and the immunoreactive protein bands are visualized, for example, in the case of use of horseradish peroxidase-conjugated anti-immunoglobulin, using enhanced chemiluminescence reagents marketed by Amersham (Bucks, United Kingdom).

To test for a neutralizing effect of antibodies generated in vivo (patients or animals), the biological activity of the peptide, protein, or protein complex of interest can, for example, be determined by using the bioassays, such, as for example, cell proliferation assays, in varying concentrations of serum of individuals or animals exposed/immunized with the peptide, protein, or protein complex of interest. Serial dilutions of the peptide, protein, or protein complex of interest are prepared in microtiter plates. Exponentially growing cells are washed and resuspended to a consistent and appropriate concentration in growth medium, and added in aliquots to each well. The plates are incubated for an appropriate period of time (depending on the cells), pulsed with vital dye for an appropriate/specified period of time, and harvested. Stained cells are counted under a microscope. For neutralization, a dilution series of serum before and after in vivo exposure (immunization) is preincubated with the peptide, protein, or protein complex of interest for an appropriate period of time before the addition of cells. Sera are preferably heat-inactivated before use in the assay.

**Retained Function**

**Functionality** - Depending on the nature of the polypeptide, protein, or protein complex, retained functionality can be tested, as examples, by comparing the functionality of the engineered material, engineered as described above, with that of the material before engineering, engineered by another method, or naturally modified by a post-translational modification that, for example, regulates the association of the polypeptides. Assays for retained functionality can be based, for example, on the physical or functional properties of the protein in *in vitro* assay systems, such as, for example, *in vitro* kinase assays. Alternatively, the engineered material can be tested for functionality by using assays based on the biological activity of the complex. For example, a growth factor complex, such as a member of the IL-8 family, can be tested for activity in chemotactic cell migration assays or beta-glucuronidase release assays (Leong SR et al., 1997. Protein Sci. 6(3): 609-17).

**Specificity** - Depending on the nature of the polypeptide, protein, or protein complex, retained specificity can be tested, as examples, by comparing the specificity of the engineered material with that of the material before engineering, engineered by another method, or naturally modified by a post-translational modification. Assays for retained specificity can be based, for example, on enzymatic substrate specificity, or ELISA-type procedures.

**Stability**

**In vitro** - Stability of the engineered material may be tested *in vitro* in, as examples, but not limited to, time-course experiments incubating the complex at varying protein concentrations and temperatures; the engineered material's stability may also be tested at various pH levels and in various redox conditions. For all of the above conditions, the remaining levels of functional complexes is determined by assaying as described above (Functionality, Stability).

**In vivo** - Pharmaceutical applications are best tested *in vivo*. *In vivo* stability of the material may be tested in, as examples, serum, incubating the engineered material in time-course experiments at various temperatures (e.g. 37°, 38°, 39°, 40°, 42°, and 45°C) and at different serum concentrations, and assaying for the remaining levels of functional complexes. Furthermore, stability of the material in the cytoplasm may be tested in time-course experiments in cell-lysates, lysed under various conditions (e.g. various concentrations of various detergents) at different temperatures (e.g. 37°, 38°, 39°, 40°, 42°, and 45°C), and assaying for the remaining levels of functional polypeptides, proteins, or protein complexes. More directly, stability of the material in the cytoplasm may be tested in time-course experiments by scrape-loading tissue culture cells with engineered material and assaying for the remaining levels of functional complexes.

**Biodistribution** - To determine the utility of an engineered polypeptide, protein, or protein complex more directly, biodistribution and/or other pharmacokinetic attributes may be determined. In a specific embodiment engineered material may be injected into a model organism and assayed for by tracing a marker, such as, for example, but not limited to, ¹²⁵I or ¹⁸F radio labels (Choi CW et al, 1995. Cancer Research 55: 5323-29), and/or by tracing activity as described above (Colcher D et al., 1998. Q.J. Nucl. Med. 44(4): 225-41). Relevant information may be obtained, for example, by determining the amount of functional material that can be expected to be pharmaceutically active due to its penetration of the specifically targeted tissue, such as, for example, a tumor. Half-life in circulation and at the specifically targeted tissue, renal clearance, immunogenicity, and speed of penetration may also be determined in this context.

**Animal And Clinical Studies** - The most conclusive measurements with regard to a conjugate's utility determine its pharmacological activity directly, either in animal studies or clinically. In a specific embodiment, such measurements may include, for example, measurements with which tumor pro- or regression is monitored upon treatment of an animal model or one or several patients with engineered material designed as an anticancer pharmacological agent. In another embodiment, such measurements may include, for example, measurements, of bone mass, such as x-ray measurements, upon treatment of an animal model or one or several patients with engineered material designed as an antimenopausal bone-loss pharmacological agent.

**Software For Residue Selection Process** - herein described is software that permits automated selection of suitable residues at which a polypeptide, protein, or protein complex may be modified. Such software can be used in accordance with the selection process taking antigen processing into account, as described above, and with geometrical, physical, and chemical criteria, such as set forth in the US patent application Stabilized Proteins (Marshall CP et al., US patent application No. 09/837,235; see especially Identification of Suitable Residue Pairs for the Reaction, Software for the Residue Selection Process in Section 5, and the Residue Pair Selection Flowchart in Section 6).

For the purposes of this invention, "administration" means any of the standard methods of administering a pharmaceutical composition known to those skilled in the art. Examples include intravenous, intraperitoneal or intramuscular administration, in vitro gene therapy via adenoviral vector or other vector (liposome), ex vivo gene therapy, oral, inhalation, etc. The administering of the proteins, protein complexes or polypeptides produced by the method of the invention may be carried out via injection, oral administration, or topical administration. the subject may be a mammal, e.g., a mouse or a human. Preferably, the mammal is a human. Alternatively the "introducing" or administering may be carried out by a means selected from the group consisting of adenovirus infection, liposome- mediated transfer, topical application to the cell, and microinjection. In another alternative, the carrier is an aqueous carrier, a liposome, or a lipid carrier.

As used herein "nucleic acid molecule" includes both DNA and RNA and, unless otherwise specified, includes both double-stranded and single-stranded nucleic acids. Also included are hybrids such as DNA-RNA hybrids. Reference to a nucleic acid sequence can also include modified bases as long as the modification does not significantly interfere either with binding of a ligand such as a protein by the nucleic acid or Watson-Crick base pairing.

Two DNA or polypeptide sequences are "substantially homologous" when at least about 80% (preferably at least about 90%, and most preferably at least about 95%) of the nucleotides or amino acids match over a defined length of the molecule. As used herein, "substantially homologous" also refers to sequences showing identity to the specified DNA or polypeptide sequence. DNA sequences that are substantially homologous can be identified in a Southern hybridization, experiment under, for example, stringent conditions, as defined for that particular system. Defining appropriate hybridization conditions is within the skill of the art. See, e.g., Sambrook et al., supra; DNA Cloning, vols I & II, supra; Nucleic Acid Hybridization, supra.

A DNA "coding sequence" or a "nucleotide sequence encoding" a particular protein, is a DNA sequence which is transcribed and translated into a polypeptide in vivo or in vitro when placed under the control of appropriate regulatory sequences. The boundaries of the coding sequence are determined by a start codon at the 5'-(amino) terminus and a translation stop codon at the 3'-(carboxy) terminus. A coding sequence can include, procaryotic sequences, cDNA from eucaryotic mRNA, genomic DNA sequences from eucaryotic (e.g., mammalian) sources, viral RNA or DNA, and even synthetic nucleotide sequences. A transcription termination sequence will usually be located 3' to the coding sequence.

"Operably linked" refers to an arrangement of nucleotide sequence elements wherein the components so described are configured so as to perform their usual function. Thus, control sequences operably linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

A cell has been "transformed" by exogenous DNA when such exogenous DNA has been introduced inside the cell membrane. Exogenous DNA may or may not be integrated (covalently linked) into chromosomal DNA making up the genome of the cell. In procaryotes and yeasts, for example, the exogenous DNA may be maintained on an episomal element, such as a plasmid. In eucaryotic cells, a stably transformed cell is generally one in which the exogenous DNA has become integrated into the chromosome so that it is inherited by daughter cells through chromosome replication, or one which includes stably maintained extrachromosomal plasmids. This stability is demonstrated by the ability of the eucaryotic cell to establish cell lines or clones comprised of a population of daughter cells containing the exogenous DNA.

As used in this specification and the appended claims, the singular forms "a," "an" and "the" include plural references unless the content clearly dictates otherwise.

This invention is illustrated in the Example sections that follow. These sections are set forth to aid in an understanding of the invention.

**Examples and Experimental Details of the Invention**

The following examples illustrate certain variations of the methods of the invention toward reducing immune responses to proteins. These examples are presented by way of illustration.

**Interleukin-2**

Several polypeptides and polypeptide complexes with significant therapeutic and commercial value have been identified in recent years. In the following section, methods of reducing immune responses to one such polypeptide, Interleukin 2 (IL-2), for which an abundance of data is available, are described in detail. Specifically, described below are the selection process of where to target a suitable modification, a dityrosine bond (see below), the introduction of point mutations, bacterial expression of the polypeptides and their purification, adjustment of the cross-link reaction conditions, the cross-link reaction itself, and analysis of the resulting protein.

**IL-2 Biology** - Interleukin 2, originally termed T cell growth factor (TCGF), was the first cytokine to be isolated, purified, and characterized at the molecular level. Structurally, IL-2 is related to many interleukins, hematopoietic cytokines, and certain other peptide- or protein hormones, such as growth hormone and prolactin. It is a low molecular weight, 15 kDa globular glycoprotein, composed of four amphipathic antiparallel helices (Robb RJ & Smith KA, 1981. Mol Immunol.18, 1087-94; Taniguchi T et al., 1983. Nature 302: 305-10.). Only antigen-activated T cells produce IL-2, and cells that respond to IL-2 are limited to: (A) Antigen-activated T- and B-cells and (B) Natural killer (NK) cells.

IL-2 promotes the proliferation, differentiation, and survival of both T- and NK cells. *In vitro,* IL-2 regulates the rate, magnitude, and duration of the proliferative expansion of antigen-activated T cells (Cantrell DA and Smith KA, 1984. Science 224: 1312-16.). *In vivo,* IL-2 is responsible for the clonal expansion of antigen-activated CD4+ (Th1 & Th2) and CD8+ T cells. Following this expansion, differentiated function of both T helper cells (CD4+) and T cytolytic cells (CD8+) remains IL-2 dependent. Moreover, the maintenance/survival of expanded T-cell clones - and thus T cell memory - is IL-2 dependent (Schorle et al., 1991. Nature 352: 621-24.; Sadlack et al., 1993. Cell 75: 253-61.; Sadlack et al., 1995. Eur. J. Immunol. 25, 3053-59).

*In vitro,* IL-2 supply circumvents apoptosis, and, analogously, IL-2 administration *in vivo* after antigen prevents the loss of expanded antigen-reactive cells (Kuroda K et al., 1996. J. Immunol. 157: 1422-31). These findings are important when considering the use of IL-2 as an immunotherapeutic, or as an adjuvant for vaccines.

IL-2 activity is mediated by specific receptors, which are expressed as follows:
- High-affinity IL-2Rs, comprised of hetero-**tri**-meric αβγ chains (kD: 10⁻¹¹), are expressed transiently on the surface of antigen-activated T- and B-cells, and 10% of NK cells;
- Intermediate-affinity IL-2Rs, comprised of hetero-**di**-meric βγ chains (kD: 10⁻⁹), are expressed constitutively on the surface of most NK cells

Thus, IL-2 concentrations of less than100pM saturate high affinity heterotrimeric IL-2 receptors, whereas an IL-2 concentration of10nM saturates intermediate-affinity IL-2 receptors (Smith KA, 1993. Blood 81: 1414-23).

**IL-2 as a Therapeutic Agent**

IL-2 was the first interleukin to be used therapeutically, and is currently used as a therapeutic agent that boosts the immune system for the treatment of cancer. Recombinant IL-2 is available as Proleukin from the Chiron Corporation, Emeryville, CA, USA, and is approved for the treatment of renal cell carcinoma and malignant melanoma. This product is produced in E. coli and differs from natural IL-2 in that it (i) is nonglycosylated, (ii) is missing the N-terminal alanine, and contains a C125A point mutation. Animal models suggested a dose-dependent effect of IL-2, which led to the development of the high-dose regimen by the NCI (Rosenberg SA et al., 1985. J. Exp. Med. 161: 1169-88). This high-dose bolus IL-2 regimen was approved by the FDA first for metastatic renal cell carcinoma in 1992 and, more recently, for metastatic melanoma in 1998. The schedule, dose, and route of administration are critical determinants of therapeutic activity of IL-2.

In the treatment of melanomas, the results of several clinical trials demonstrate that high dose IL-2 therapy (up to five courses, 6-12 weeks apart) yield an objective response rate of 16%, with a 4% durable complete response rate. Importantly, 28% of the responding patients, including 59% of the patients achieving a complete response, have remained progression free at a median follow-up of 62 months, suggesting that some patients may have been cured (Atkins MB et al., 1999. J Clin Oncol 17: 2105-16.). High dose IL-2 therapy of individuals suffering from renal cell carcinoma results in an antitumor response in about 15%, with about 5% of individuals achieving a long-term, complete response, a significant survival benefit for a subset of patients (Bleumer I et al., 2003. Eur. Urol. 44: 65-75).

The precise mechanisms of these antitumor responses remain obscure. However, a broad and increasing range of proteins and their component peptides recognized by host T cells of patients with melanoma in the context of the MHC has been defined. (Bakker ABH et al., 1994. J Exp Med. 179: 1005-09; Wolfel T et al., 1994. Eur J Immunol. 24: 759-64). A number of groups have conducted clinical trials of peptides from antigens such as gp100, MART-1/Melan A, and tyrosinase. (Marchand M et al., 1995. Int J Cancer. 63: 883-85; Traversari C et al., 2002. Clin. Oncol. 20(8): 2045-52; van der Bruggen P et al., 1994. Eur J Immunol. 24(12): 3038-43; Zhai Y et al., 1996. J Immunol. 156 (2): 700-10; Cormier JN et al., 1997. Cancer J. Sci. Am. 3(1): 37-44), and IL-2 is currently in clinical development as an adjuvant for therapeutic cancer vaccines with promising results. For example, in a small phase II trial for the treatment of metastatic melanoma, immunization with a modified MART-1 peptide (vaccine antigen) was combined with high dose bolus IL-2 in 19 patients, of which eight achieved an objective response - a response rate of 42% (Rosenberg SA et al., 1998. Nat. Med. 4: 321-7).

**IL-2 Structure**

Cytokines fall into only a few structural classifications. The family of Short-chain 4 Alpha-helical Bundles includes but is not limited to, colony stimulating factors M-CSF and GM-CSF, IL2, IL3, IL4, IL5, IL7, IL9, IL13, SCF, and IFN-γ, and the family of Long-chain 4 Alpha-helical Bundles includes, but is not limited to erythropoietin, IFN-α, IFN-β, growth hormone, G-CSF, IL6, IL10, IL11, IL12 alpha, PRL, CNTF, LIF, OSM. Within the family of Long-chain Beta-Sheets, Jelly Rolls - that generally trimerize, bind three receptor subunits, and are often cell surface bound - include, TNA a, and -b, 4-1BB-L, APRIL, BAFF, CD27L, CD30L, CD40L, FasL, LIGHT, Ox-40-L, TRANCE, TRAIL, AND TWEAK; Beta-trefoils include, but are not limited to, IL1a and b, ac.FGF, bas.FGF, INT-2, and KGF; and Cystine Knots - a large family of cytokines that generally homodimerize and contain three disulfide bonds - include, TGFβ1, 2, and 3, activin, inhibin, the BMP's (more than 30), PDGF a and b, VEGF, PIGF, NGF, BDNF, NT3, and NT4/5. Short-chain alpha/beta cytokines include, the EGF-domain, EGF, TGF-α, beta-cellulin, SCDGF, CCGF, Amphiregulin, and HB-EGF. Chemokines (C-C, C-X-C, and C-XXX-C; also classified as α+ structures) include, MCP-1, -2, and -3, RANTES, MIP1-α and -β, IL-8, GRO, PF-4, MIP-2, NAP-2, GCP-2, ENA-78, and IP-10. The Insulin-like cytokines include, insulin, IGF I and II, relaxin, and bombyxin. Some cytokines have mosaic structures, including, HGF, IL12, Ig-EGF-TK-Cyt, the HRG alphas and betas, NDF, ARIA, and GGF.

IL2 belongs to the family of Short-chain Alpha-helix Bundles, and its structure comprises a bundle of 4 helices (termed A-D), flanked by 2 shorter helices and several poorly defined loops (see Figure 5). Secondary structure analysis has suggested particular similarity to IL4 and granulocyte-macrophage colony stimulating factor (GMCSF).

Many cytokines have similar structures and share receptors, and yet vary greatly in function, as, for example, homologous cytokines may differ considerably in their mode of interaction with a shared receptor. Type I interferons (IFN-α/β) illicit pleiotropic biological activities. These different IFN subtypes activate the same cell surface receptor complex to mediate variable responses. Differences in critical amino acid residues among the IFN-αs and IFN-β determine the nature of the ligand-receptor interaction and their biological responses.

The high affinity receptor for interleukin-2 (IL-2) contains three subunits called IL-2R α, β and γ. The low affinity receptor consists only of the IL-2R α and β. Mutagenesis studies on both human and mouse IL-2 demonstrated that residues located in the AB-loop, B-helix, CD-loop and D-helix are involved in interactions with the α subunit of the IL2 receptor. Earlier studies showed that there are 44 residues in mouse IL-2 (which is 65% homologous with human IL-2) that are assigned functional importance from mutational analysis. 21 of these residues are structural; 19 residues (from three regions in the protein structure) are important for IL-2R alpha interaction; three residues (from two regions in the protein structure) are important for IL-2R beta interaction; and a single residue is important for IL-2R gamma interaction (Zurawski SM et al., 1993. EMBO J. Dec 15;12(13):5113-9).

**Immune Responses to IL-2**

Many cytokines are reported to elicit humoral immune responses when administered as therapeutic agents, including interferon alpha, beta, IL-11, growth hormone, insulin, GM-CSF, BMP-7, and PEGylated MDGF, Factors VIII and IX, several therapeutic monoclonal antibodies, fusion proteins, and therapeutic enzymes (Schellekens H, 2001. Presentation given at the October 31st through November 2nd, 2001 IABs international meeting: http://www.iabs.org/Page121.html; Bordens R, 2001. Presentation given at the October 31st through November 2nd, 2001 IABs international meeting. http://www.iabs.org/Page121.html.; Prummer O, 1997. Biotherapy 10(1):15-24). Immune responses to biopharmaceuticals have more recently become a major focus of the FDA and biopharmaceutical industry, as in many cases they have been shown to lead to:
- Partial and total loss in biotherapeutic efficacy;
- Immune complex disease;
- Cross reactive responses to endogenous proteins that can in some cases lead to severe, life threatening disorders, such as red blood cell aplasia (Epogen) or thrombocytopenia (MGDF).

IL-2 is reported to elicit the production of neutralizing anti-IL-2 antibodies that impair its efficacy as a biotherapeutic. The rate of recombinant IL-2 treatment-induced anti-IL-2 antibodies exceeds 50%. The duration of IL-2 treatment, cumulative doses, and the route of administration are likely to determine the rate of seroconversion, and the composition and properties of the anti-IL-2 antibodies. Anti-IL-2 antibodies frequently react with both recombinant and natural IL-2 types, and limit the expression of IL-2-dependent proteins *in vivo* (Prummer O, 1997. Biotherapy 10(1):15-24).

Prolonged treatment with more easily tolerated low dose regimens of IL-2 may lead to the production of higher titer, neutralizing antibodies that cross-react with endogenous IL-2, and render patients dependent on exogenous IL-2 supply.

**ADVANTAGES OF THE TYROSYL-TYROSYL CROSS-LINK**

The essence of dityrosine (DT) crosslinking chemistry is shown in Figure 3, where a highly stable, covalent C-C bond is the result of an oxidative crosslink reaction between two tyrosine residues. These tyrosines must be situated in close proximity to each other, either within a single folded polypeptide chain, or on closely interacting protein domains within a complex. Because proximity of the tyrosyl side chains is a prerequisite to bond formation (Brown K et al., 1998. Biochemistry 37(13):4397-406), and because no atom is added in the formation of these bonds, the resulting "staples" are non-disruptive to the protein structure.

Disulfide bonds (DS bonds) have also been found in many eukaryotic proteins of diverse function; these bonds can form spontaneously. Intra-molecular S-S crosslinks often stabilize protein domains, and inter-molecular S-S bonds provide stability for the quaternary structure of many protein complexes. As a protein engineering tool, the introduction of disulfide bonds can provide an impressive degree of stabilization of proteins without impairing their activity or specificity. For example, stabilizing immunoglobulin Fv fragment complexes using disulfide bonds is one of the most effective technologies for that purpose. However, disulfide bonds are inherently labile, and bacterial production of native, biologically active eukaryotic proteins is difficult for disulfide-bonded and multisubunit proteins (Mantile G et al., 2000. Biotechnol Prog. 16(1):17-25.). Therefore, disulfide bonds have not been used in biopharmaceutical development as broadly as otherwise would have been warranted given the tremendous advantages of non-peptide covalent bonds in protein engineering. Dityrosine bonds, on the other hand, are extremely stable. DT interference with protein folding is not anticipated, as DT - as opposed to DS bonds - do not form spontaneously. Furthermore, DT bonded protein have been shown to retain impressive levels of activity: Subtilisin E - 80% (data not shown); RNase A - 100%, chymotrypsin - 50% (Aeschbach et al., 1976. Biochim Biophys Acta 439: 292-301), lactoperoxidase -100% (Lardinois et al., 1999. J Biol Chem. 274(50): 35441-8).

DT bonds are ubiquitously present in human proteins. For example, DT bonds are introduced into the structure of human periodontal ligament collagen through a peroxidase-catalyzed physiological pathway (Tenovuo J & Paunio K, 1979. Arch. Oral Biol. 24,591; Tenovuo J & Paunio K, 1979. Acta Odontol Scand. 37(3): 147-52). More interestingly, DT bonds have also been shown to form the lattice structure of wheat gluten - the quaternary protein structure comprising the glutenin subunits - e.g. in bread dough during mixing and baking (Tilley KA, 2001. J. Agric. Food Chem 49, 2627). Thus, bonded tyrosine amino acids are very likely (1) not to be toxic, and (2) not to cause immune responses.

It is known that engineering biopharmaceuticals can cause severely detrimental immune responses. These effects range from neutralizing the therapeutic potential and efficacy of drugs, to immune complex disease, and in several instances now to life-threatening disorders, such as red blood cell aplasia and thrombocytopenia, due to cross reactivity of anti-biopharmaceutical antibodies with endogenous protein (Epogen and MGDF, respectively).

That DT bonds don't elicit immune responses is supported by their above described presence in gluten - a protein component of a major staple in many human's diet- and other endogenous proteins in human. Furthermore, in the context of protein therapeutics, DT crosslinks are unlikely to yield T-cell epitopes that lead to MHC restricted antigen presentation on the surface of APCs, T helper cell activation, and the induction of humoral immune responses, as they are stable under conditions of endosomal and lysomal vesicles, and structurally incompatible with MHC presentation. DT bonded di-peptides' incompatibility with MHC presentation is independent of the heterogeneity of HLA molecules that provides the immune system with its ability to recognize a very wide range of epitopes.

Summary of the major advantages of directed DT crosslinking:
- Stability of the linkage: The resulting C-C is stable under virtually all physiological conditions, including the environments of all segments of the GI tract and of the endocytic and lysosomal vesicles of antigen presenting cell (APCs).
- Structurally and Functionally Non-disruptive: Because proximity of the tyrosyl side chains is required for DT bond formation (Brown *et al.,* 1998), and because no atom is added in the formation of these bonds, the resulting crosslinks are non-disruptive to protein structure and function. DT interference with protein folding is not anticipated, as DT do not form spontaneously. Several DT bonded proteins have been shown to retain impressive levels of activity.
- Non-toxic: DT bonds are present in large quantities in one of the most common foods, wheat-based bread, and DT bonded proteins are present in human. Thus, DT bonds and DT bonded proteins are very likely non-toxic.
- Non-immunogenic:
- T-cell epitopes: DT bonded di-peptides resulting from antigen processing (see Figure 1) are structurally incompatible with MHC presentation, and therefore it is very unlikely that DT bonds will introduce novel T-cell epitopes that lead to T-helper cell, and subsequent B-cell activation and antibody production.
- B-cell epitopes: Furthermore, DT bonds are present in large quantities in sone of the most common human foods, and DT bonded proteins are present in human. Thus, DT bonds are likely known to the immune system as self, and unlikely to provide B-cell epitopes that lead to B-cell activation and the production of harmful antibodies.

There are two key elements to the introduction of dityrosine bonds: (i) the ability to design the placement of two tyrosines in sufficient proximity, and (ii) the ability to achieve reduced immune responses after performing the reaction.

An investigation focused on the above two points using the protease subtilisin E as a model enzyme, which is a highly promiscuous methionine protease. Using the selection methodology described in a prior patent application (Stabilized Proteins, see Marshall *et al.,* U.S. Serial No. 09/837,235, filed April 18, 2001 and U.S. Serial No. 60/159,763, filed October 15, 1999, and PCT International Application No. PCT/US00/28595, filed October 16, 2000), candidate residues for DT crosslinking were identified. The targeted residues were mutated to tyrosine, and the protein was expressed, purified, and subjected to crosslinking according to standard procedures known to one skilled in the art. The formation of the DT bond in K237Y was confirmed by fluorescent spectroscopy, and the DT crosslinked protease maintained approximately 80% of the enzymatic activity of the wild type protein (data not shown). Inactivation of the protein was shown to be impaired (see Figure 2). No other alterations to the protein structure were observed by LC-MS. Additional peaks were not observed, nor were broadening of the main peaks (see Figure 4).

These data demonstrate that DT bonds can be targeted within the structures of proteins without otherwise altering the structure of the protein while maintaining a high degree of the uncrosslinked protein's activity, and, given that subtilisin E is inactivated by autodegradation, impair proteolytic degradation of a substrate. Thus, it is likely that antigen processing, which involves partial proteolytic degradation of protein antigen, can also be inhibited, and that it is possible to reduce or prevent immune responses by blocking this pathway toward MHC display of immunogenic peptides, and T-cell activation.

**RESIDUES FOR TYROSYL-TYROSYL CROSS-LINK**

Optimal residues, to which the cross-link reaction is directed, are selected based on structural measurements that assess the following issues:
- will the dityrosine bond form: i.e. are the tyrosyl side chain in close proximity
- will the protein remain structurally and functionally intact upon introduction of the required (one or two) point mutations to tyrosine and crosslinking;

**Table 3. Potential residue pairs for the tyr-tyr cross-link.**

| Residue pairs | | | Cα-Cα distance | Δ v.d. Waals | Δ Hydrophobicity |
|---|---|---|---|---|---|
| Tyr | 107 Tyr | 45 | 5.86 | 0 | 0 |
| Tyr | 107 Pro | 47 | 4.11 | 51 | 0.14 |
| Thr | 111 Tyr | 45 | 5.07 | 48 | 0.31 |
| Asn | 29 Phe | 78 | 5.18 | 51 | 0.10 |
| Tyr | 107 Met | 46 | 5.94 | 17 | 0.38 |
| Tyr | 107 Lys | 48 | 5.65 | 6 | 1.76 |
| Phe | 78 lle | 28 | 5.18 | 23 | 2.08 |
| Thr | 113 Lys | 43 | 5.04 | 54 | 2.07 |
| Leu | 40 Thr | 113 | 5.52 | 65 | 0.53 |
| Ile | 28 Phe | 78 | 5.18 | 23 | 2.08 |
| Thr | 113 Phe | 44 | 5.88 | 54 | 0.63 |
| Thr | 113 Leu | 40 | 5.52 | 65 | 0.53 |
| Asp | 20 lle | 86 | 6.09 | 67 | 0.02 |
| Asn | 90 Leu | 59 | 6.14 | 62 | 0.20 |
| Ile | 28 Asn | 77 | 6.31 | 62 | 0.10 |
| Phe | 117 Met | 46 | 6.3 | 23 | 1.32 |
| Ala | 112 Lys | 43 | 5.11 | 80 | 1.40 |
| Ala | 112 Phe | 44 | 5.15 | 80 | 1.30 |
| Leu | 117 Ile | 89 | 5.94 | 34 | 1.98 |
| Thr | 111 Lys | 43 | 5.72 | 54 | 2.07 |
| Ile | 128 Thr | 51 | 5.91 | 65 | 0.83 |
| Asp | 20 Leu | 85 | 6.09 | 67 | 0.32 |
| Thr | 111 Phe | 44 | 6.31 | 54 | 0.63 |
| Leu | 21 Ile | 122 | 6.36 | 34 | 1.98 |
| Ser | 87 Glu | 60 | 5.93 | 100 | 1.49 |
| Asn | 29 Asn | 77 | 6.31 | 90 | 2.08 |

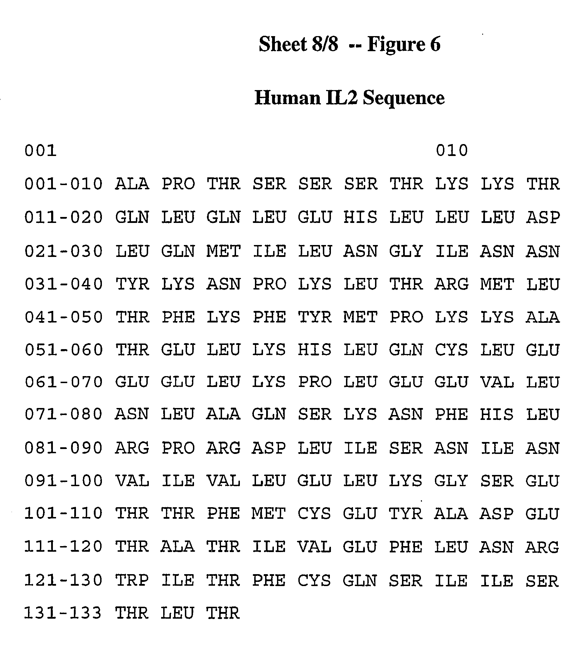

Given the relatively small number of amino acids in the structure of IL-2 (see Figure 6), it is the most efficient approach to identify the set of residue pairs with alpha carbon distances less than 6.5 Angström (omitting residue pairs that contain glycines), to generate the point mutants, crosslink, and test the crosslinked protein constructs for retained activity and immunogenicity, as described in detail below. Table 3 contains a set of 26 residue pairs in the structure of IL2 that are appropriate for this set of experiments. For each residue pair, the alpha carbon distance and the difference between the van der Waals volumes and hydrophobicity of the existing amino acids, and the van der Waals volumes and hydrophobicity of tyrosine is calculated to rank the constructs in their likelihood to retain structure and function upon the introduction of the point mutation to tyrosine.

Given the proximity of Tyr107 and Tyr45, it is possible that wild type IL2 already forms a DT bond upon exposure to crosslinking conditions. If this is the case, the DT bonded construct is evaluated for retained activity and immunogenicity before point mutations are made. If this bond is found to be undesirable, both of Tyr107 and Tyr45 are mutated to Phe in separate constructs, and the point mutants are made in the constructs already containing these Tyr to Phe mutations for further evaluation.

**Molecular Biology & Protein Expression**

**Constructs & Expression systems** - IL-2 protein constructs are expressed as (his)₆-tagged constructs with a TEV cleavage site that allows removal of the tag for *in vivo* experiments to facilitate purification. Biochemical and *in vitro* experimentation do not require the removal of the tag. Preferably, a his-tagged human IL-2 gene in a bacterial expression plasmid is used (pET vector, Novagen, Madison, WI) that directs expression of IL-2 at high levels in E coli BL21(DE3).

**Point Mutations** - The residues selected for mutation to tyrosine by structural analysis based on structure/function (see above) analyses (and computational modeling may also be applied) are mutated using the QuikChange^{™} Site-Directed Mutagenesis Kit (Stratagene, Catalog # 200518). If tyrosine residues are identified that will form undesirable DT bonds (e.g. if Tyr45 and Tyr 107 form a bond that impairs activity), one or each of the pair is mutated to phenylalanine, and the construct is assayed for retained activity (see below).

**Protein Expression** - IL-2 protein expressed at high levels in E coli BL21(DE3) forms inclusion bodies, and the protein is refolded using urea denaturation followed by gradual dialysis (Wilson D, PromAb, personal communication).

**Protein Purification** - Protein constructs is taken up, or dialyzed into, 50 mM PBS, 1mM Imidazole pH 7.5 and applied on a Ni-NTA-His-Bind^{™} resin column (Novagen). After extensive washing with essentially the same buffer (10 mM imidizole), protein is eluted with 200 mM Imidazole pH 7.5, dialyzed overnight, and stored frozen. Protein purity is determined by SDS-PAGE and silver staining.

**DT Crosslinking and Purification** - Once the expression system is established and the recombinant constructs are obtained and purified, the proteins are crosslinked under optimized conditions (see Marshall et al., U.S. Serial No. 09/837,235, filed April 18, 2001 and U.S. Serial No. 60/159,763, filed October 15, 1999, and PCT International Application No. PCT/US00/28595, filed October 16, 2000). Crosslinking is carried out either enzymatically, using commercially available peroxidases, or chemically. To optimize the conditions for each construct several parameters will be varied. For example, variation/adjustment of the pH of the reaction buffer, followed by the addition of water miscible organic solvents, is likely to have the most pronounced effect on the efficiency. Two chemical methods, i.e. chloro-mangenese (MnIII)-tetra(p-sulpfonato)phenyl porphyrine (MnTPPS) + KHSO₅ and ruthenium (II) tris-bypyridyl +(NH₄)₂S₂O₈), will also be used in these experiments. The best performing method/set of experimental conditions will be adopted for the preparation of the DT IL-2 constructs.

Due to the distinct fluorescent properties of DT bonds, and the sensitivity of the assay, detection of as little as 0.1 mol of DT per mol of enzyme is straightforward. Fluorescence-based assays are easily translated into high throughput formats, e.g. using a 96-or 384-well plate reader, and therefore optimization of the reaction conditions, and evaluation of a relatively large number of catalyst/oxidant pairs are straightforward in this investigation. The ability to follow the formation of DT bonds with a sensitive and simple fluorescence-based high through-put screen facilitates the generation of optimally engineered constructs and, more importantly perhaps, rapidly eliminates early non-productive mutations. Fluorescence is analyzed at 280 and 320nm excitation wavelengths with emission maximum of 310 and 410 nm for tyrosine and DT, respectively. Neither tyrosine, nor tryptophan residues interfere with the fluorescence of DT (Kanwar & Balasubramanian, 2000. Biochemistry 39: 14976; Malencik DA & Anderson SR, 1994). Biochemistry 33: 13363-13376). Crosslinked protein is purified by standard methods, e.g. gel-filtration HPLC.

**Analysis of DT IL-2 constructs**

**Protein Integrity and Retained Activity**

**Amino acid analysis** - Cross-linked constructs are analyzed by LC-MS to confirm the integrity of the protein. The same MS technique is also be applied toward confirmation of DT bond formation at the predicted location within the protein structure by partial proteolytic digestion and LC-MS analysis of the resultant fragments.

The results of this analysis are further verified by full protein hydrolysis (6N HCl, 24h). Pre-column modification of amino acids with e.g. 4'-dimethylaminoazobenzene-4-sulfonyl (dabsyl) chloride, followed by reverse phase HPLC allows the separation and quantification of di-tyrosine along side all other constituent amino acids, after calibration with appropriate standards (Malencik DA et al., 1990. Anal Biochem., 184, 353-359).

Furthermore, amino acid analysis of crosslinked and uncrosslinked protein complex is performed by complete hydrolysis of the protein (6N HCl, 1% Phenol, 110°C for 24 hrs), and subsequent LC-MS analysis according to standard, published procedures (e.g. Tilley KA, 2001. J. Agric. Food Chem 49, 2627).

**Tissue Culture Evaluation of Retained Activity** - The IL-2 bioassay is the prototypic *in vitro* cytokine assay. Splenocytes obtained from Balb/C mice are incubated at a concentration of 5x10⁶/ml, in the presence and absence IL-2 activity in 96-well microtiter plates, and cultured overnight at 37°C. The experimental samples are controlled by comparison with the standard curve, using the EC ₅₀ for comparison. The assay is sensitive to a concentration of 1 pM, and the IL-2 dose-response curve occurs between 1 and 100 pM. The International Standard Specific Activity of purified, homogeneous IL-2 is defined as 15 million IU/mg IL-2 protein, and 1 IU/mL=4.5 pM (Gillis S et al., 1978. J. Immunol. 120, 2027-32; Robb RJ et al., 1981. J. Exp. Med. 154: 1455-74.).

Several concentrations of control (Proleukin) and DT IL-2 constructs are added, ranging from 5 pM to 50 pM, and the proportion of cells synthesizing DNA will be quantified by vital dye uptake (Gillis S et al., 1978. J. Immunol. 120, 2027-32) and plotted.

**Pharmacokinetic Analysis** - Balb/c mice are given s.c. injections of control and DT IL-2 at dosages of 10,000, 50,000, and 250,000 IU/m², and biodistribution and pharmacokinetic analyses are performed to establish the rate of adsorption, vascular retention, half-life of the construct in serum, and clearance rate of the construct by comparison to control.

All *in vivo* studies are carried out in Balb/C mice, male and female equally, 6-8 weeks of age (e.g. Harlan Sprague Dawley). The mice are given control IL-2 and DT IL-2 at dosages of 10,000, 50,000, and 250,000, and 1,000,000 IU/m² through s.c. injections in 50µl volumes from a 30-gauge needle. For the purpose of this study, an IU for both constructs is based on the definition of 1 IU of IL-2 = 4.5 pM. Ether is used for anesthesia; the animals are given free access to food and water.

Blood is drawn in standard time-course experiments, from 0 to 144 h, and repetitive blood draws are performed on five mice per treatment group for each assay time. Each blood sample is stored on ice prior to centrifugation (12 000 g, 5 min, 4 °C), and serum is stored a.t -20 °C until assayed.

Detection of IL-2 in serum is carried out using a commercially available human IL-2 ELISA (e.g. Immunotech, France) according to the manufacturer's recommended procedures. This kit provides microtiter plates coated with a mouse monoclonal anti-(human IL-2) antibody. A second anti-human IL-2monoclonal antibody linked to reporter enzyme quantifies the bound IL-2. After washing, bound enzymatic activity is measured with a colorometric substrate, also provided in the kit. Both anti-human IL-2 antibodies are initially tested for their ability to bind the DT IL-2 construct.

Anti-human IL-2 Westernblot analysis is performed to monitor for proteolytic degradation of the IL-2 constructs *in vivo.* A commercially available anti-IL-2 antibody is used that recognizes the DT IL-2 constructs, and that is specific for human IL-2.

T-cell proliferation assays are performed to confirm the biological activity of control *in vivo* (Proleukin and wt IL-2 expressed and purified in the same system as the DT IL-2 constructs) and DT IL-2 protein (see above) after serum treatment and recovered in mouse serum. Control Il-2 and DT IL-2 construct protein is incubated in mouse serum (Sigma) at a concentration of 10,000 units per ml, and incubated for periods ranging from 0-144 hrs at 37°C. 1:10 and subsequent serial dilutions of the serum in tissue culture medium will be tested for their ability to induce proliferation of CTLLs. Serum collected from injected mice will also be diluted in tissue culture medium and analyzed for its ability to induce CTLL proliferation.

As a proxy for systemic inflammatory response syndrome (SIRS), or vascular leakage syndrome (VLS), the proinflammatory cytokines TNFα, Infy, and GM-CSF in serum are quantified before and after administration of control IL-2 (Proleukin and wt IL-2 expressed and purified in the same system as the DT IL-2 constructs) and DT L-2 at the above concentrations.

For pharmacokinetic data analysis, assay times are calculated as the time from injection to collection. t_{1/2} calculations are performed using a standard software package. Serum concentration is plotted against time after s.c. injection by non-linear regression. The half-lives and area under the curve (AUC) are calculated as described in "Cancer chemotherapy and biotherapy: principles and practice" (Collins JM, 1996. In: Chabner BA, Longo DL (eds) Cancer chemotherapy and biotherapy: principles and practice. Lippincott-Raven, Philadelphia, p 17). The concentrations of control hIL-2 and DT IL-2 constructs at time zero are extrapolated on the basis of the calculated t_{1/2} at the first measurable assay point.

**Immune Responses to IL-2**

In the following set of experiments, immune responses to control and DT IL-2 constructs leading to the production of neutralizing and non-neutralizing antibodies are examined.

**Mouse IL-2 Treatment Regimen** - As IL-2 immunogenicity is observed in high dose treatment regimens in human, 2.4 x 10⁶ IU/m2 of control (Proleukin) and DT IL-2 construct protein is administered to male and female Balb/C mice, 6-8 weeks of age, by s.c. injection twice daily for 10 days, and the treatment cycle is repeated every fourth week, for a total of four cycles. Venous blood samples are collected in sterile tubes prior to the first injection and 3 hrs after injection on days 1 and 10 of every treatment cycle. Serum is separated and stored frozen, as described above, and serum levels of control and DT IL-2 are assayed as described above.

**Detection of anti-IL-2 antibodies**

**ELISA assay** - A solid phase indirect ELISA is used to detect binding of anti-IL-2 antibodies. Microtiter plates are incubated at 4°C overnight with 100 µl/well of IL-2 (Proleukin; 0.25 µg/ml) in 50 mM carbonate-bicarbonate buffer (pH 9.7). After three washes with PBS (pH 7.4), PBS containing 1% BSA and 0.05% Tween 20 is added to each well, and the plates are incubated for 1 h at 37°C. Serum samples are diluted 1:20 in PBS/BSA, and 100 µl of the samples are added in duplicates to the wells. The plates are incubated for 2 h at 37°C. After an additional three washes with PBS, 100 µl of alkaline phosphatase-conjugated anti-mouse antibodies (Sigma Chemical Co.) diluted 1:1000 in PBS are added to each well and incubated for 2 h at 37°C. The wells are then washed three times before the enzyme reaction at room temperature using p-nitrophenyl phosphate (1 mg/ml; Sigma Chemical Co.) in diethanolamine buffer (pH 9.8), The absorbance is read at 405 nm in an automatic ELISA reader (e.g. Multiskan PLUS; Labsystems).

**Immunoblotting of IL-2 Antibodies**. 3 µg of IL-2 protein (Proleukin) per samples/lane will be run on 12.5% acrylamide gels under reducing and nonreducing conditions, and the protein will be transferred to PVDF membranes. The membranes will be blocked using a solution of 5% (w/v) milk powder in PBS for 30 min on a rotary shaker. The blots will then be incubated with serum samples at dilutions of 1:200 and 1:1000 in PBS/milk (before and after injection regimen) and commercially available control anti-human IL-2 overnight at room temperature on a rotary shaker. The blots will be washed four times with PBS/milk, and further incubated with horseradish peroxidase-conjugated anti-mouse immunoglobulin (e.g. Sigma Chemical Co.) at a dilution of approximately 1:2000 in PBS/milk solution for 1 h on a rotary shaker. The blots will be washed again four times with PBS/0.05% Tween 20, and the immunoreactive protein bands were visualized using the enhanced chemiluminescence reagents (Amersham. Bucks, United Kingdom).

**CTLL Proliferation Assay for Detection of Neutralizing IL-2 Antibodies.** The biological activity of IL-2 is determined using the CTLL proliferation bioassay, as described above. Serial dilutions of IL-2 (Proleukin) will be prepared in 50-µl volumes in 96-well microtiter plates. Exponentially growing CTLL cells are washed and resuspended to a concentration of 100/ml in RPMI 1640 containing 10% FCS, and added in 50-µl aliquots to each well. The plates are incubated for 24 h, pulsed for 4 h with vital dye, and harvested, and the stained cells are counted under a microscope. For neutralization, a 2-fold dilution series giving a final dilution of 1:20 to 1:2560 of mouse serum before and after treatment regimen is preincubated with IL-2 (2 IU/ml) for at least 1h before the addition of cells. Sera will be heat-inactivated at 56°C for 30 min before use in the assay.

## Claims

1. A method for reducing the immunogenicity of a peptide, protein or protein complex comprising:
(1) identifying or selecting at least one immunogenic epitope of the peptide, protein or protein complex to be cross-linked; and
(2) introducing at least one cross-link between two amino acids in the peptide, protein or protein complex, wherein the cross-link is stable or irreversible in vivo, and wherein the cross-link reduces or inhibits the immunogenicity of the at least one epitope compared to the immunogenicity of the at least one epitope in an identical peptide, protein or protein complex not so cross-linked, and the cross-linked peptide, protein or protein complex is modified in such a way that the proteolytic fragments produced by antigen processing display reduced HLA-DM mediated peptide loading or peptide presentation or are sensitised to HLA-DM mediated peptide editing;
or any combination thereof, compared to an identical peptide, protein or protein complex not so cross-linked when administered to an individual.

2. The method of claim 1 wherein the epitope is selected from any peptide within the peptide or protein sequence with the ability to bind to the groove of the MHC molecule for presentation to the immune system.

3. The method of any preceding claim wherein a fold in the peptide, protein or protein complex is stabilised by the cross-link.

4. The method according to any preceding claim, further comprising introducing at least one other modification in the peptide, protein, or protein complex selected from the group consisting of point mutation, deletion of one or more amino acids, insertion of one or more amino acids, or derivatization, comprising PEGylation, glycosylation, acetylation, amidation, and formylation; or any combination thereof; wherein the at least one other modification reduces endopeptidase-mediated proteolytic degradation, HLA-DM mediated peptide loading or peptide presentation, exopeptidase-mediated proteolytic degradation, or any combination thereof; sensitises to HLA-DM mediated peptide editing; reduces T cell complex formation or T cell activation in response to the peptide, protein or protein complex, or both; reduces T cell activation in response to the peptide, protein or protein complex; reduces immunogenicity of the peptide, protein or protein complex; or any combination thereof compared to an identical peptide, protein or protein complex not so modified.

5. The method according to any of claim 1, 2 or 3 wherein the cross-linked peptide, protein or protein complex retains at least one function of the peptide, protein or protein complex in the absence of the at least one cross-link.

6. The method according to claim 4, wherein the modified peptide, protein or protein complex retains at least one function of the peptide, protein or protein complex in the absence of the at least one cross-link and the at least one other modification.

7. The method according to any preceding claim, wherein the peptide, protein or protein complex comprises a therapeutic product, a diagnostic product, an enzyme, a hormone, a receptor, a growth factor, or an antibody or a fragment thereof.

8. The method according to any preceding claim wherein at least one cross-link is selected from the group comprising an oxidative cross-link, a thiol reactive cross-link, an amine reactive cross-link, a cross-link between non-classical amino acids incorporated into the peptide, protein or protein complex, a homo-cross-link, a hetero-cross-link and a photo reactive cross-link.

9. The method according to any preceding claim, wherein at least one cross-link comprises a di-tyrosine cross-link between two amino acids in the peptide, protein or protein complex.

## Patentansprüche

1. Verfahren zur Verringerung der Immunogenizität eines Peptids, Proteins oder eines Proteinkomplexes, aufweisend:
(1) Identifizieren oder Auswählen zumindest eines immunogenen Epitops des Peptids, Proteins oder Proteinkomplexes, das quervernetzt werden soll; und
(2) Einführen zumindest einer Quervernetzung zwischen zwei Aminosäuren in dem Peptid, Protein oder Proteinkomplex, wobei die Quernetzung *in vivo* stabil oder irreversibel ist, und wobei die Quervernetzung die Immunogenizität des zumindest einen Epitops, verglichen mit der Immunogenizität des zumindest einen Epitops in einem identischen Peptid, Protein oder Proteinkomplex, das nicht so quervernetzt ist, verringert oder inhibiert, und wobei das quervernetzte Peptid, Protein oder der Proteinkomplex auf eine Weise modifiziert ist, dass die proteolytischen Fragmente, die durch Antigenentwicklung produziert werden, eine verringerte HLA-DM-vermittelte Peptid-Beladung oder Peptid-Präsentation zeigen oder empfindlich gemacht werden für HLA-vermittelte Peptideditierung, oder jede Kombination hiervon, verglichen mit einem identischen Peptid, Protein oder einem Proteinkomplex, der nicht derart quervernetzt ist, wenn er einem Individuum verabreicht wird.

2. Verfahren nach Anspruch 1, wobei das Epitop ausgewählt ist aus irgendeinem Peptid innerhalb der Peptid- oder Proteinsequenz mit der Fähigkeit, zur Präsentation an das Immunsystem an die Nut des MHC-Moleküls zu binden.

3. Verfahren nach irgendeinem der vorstehenden Ansprüche, wobei eine Falte in dem Peptid, Protein oder Proteinkomplex durch die Quervernetzung stabilisiert wird.

4. Verfahren nach irgendeinem der vorstehenden Ansprüche, ferner aufweisend das Einführen zumindest einer anderen Modifikation in das Peptid, das Protein oder den Proteinkomplex, ausgewählt aus der Gruppe bestehend aus Punktmutation, Auslöschung einer oder mehrerer Aminosäuren, Einfügen einer oder mehrerer Aminosäuren, oder Derivatisierung, aufweisend PEGylierung, Glycosylierung, Acetylierung, Amidierung und Formylierung oder irgendeine Kombination hiervon, wobei die zumindest eine andere Modifikation den Endopeptidase-vermittelten proteolytischen Abbau, die HLA-DM-vermittelte Peptidbeladung oder Peptidpräsentationsdarstellung, den Exopeptidasevermittelten proteolytischen Abbau verringert, oder irgendeine Kombination hiervon, gegenüber HLA-DM-vermitteltem Peptideditieren empfindlich macht, T-Zellen-Komplexbildung oder T-Zellen-Aktivierung als Antwort auf das Peptid, Protein oder den Proteinkomplex reduziert, oder beides, die T-Zellen-Aktivierung in Antwort auf das Peptid, Protein oder den Proteinkomplex reduziert, die Immunogenizität des Peptids, Proteins oder des Proteinkomplexes reduziert, oder irgendeine Kombination hiervon, verglichen mit einem identischen Peptid, Protein oder Proteinkomplex, der nicht so modifiziert ist.

5. Verfahren nach irgendeinem der Ansprüche 1, 2 oder 3, wobei das quervernetzte Peptid, Protein oder der Proteinkomplex zumindest eine Funktion des Peptids, Proteins oder des Proteinkomplexes in Abwesenheit der zumindest einen Quervernetzung zurückbehält.

6. Verfahren nach Anspruch 4, wobei das modifizierte Peptid, Protein oder der Proteinkomplex zumindest eine Funktion des Peptids, Proteins oder Proteinkomplexes in der Abwesenheit der zumindest einen Quervernetzung und der zumindest einen anderen Modifikation zurückbehält.

7. Verfahren nach irgendeinem der vorstehenden Ansprüche, wobei das Peptid, das Protein oder der Proteinkomplex ein therapeutisches Produkt, ein diagnostisches Produkt, ein Enzym, ein Hormon, einen Rezeptor, einen Wachstumsfaktor oder einen Antikörper oder ein Fragment hiervon aufweist.

8. Verfahren nach irgendeinem der vorstehenden Ansprüche, wobei die zumindest eine Quervernetzung ausgewählt ist aus der Gruppe, die eine oxidative Quervernetzung, eine Thiol-reaktive Quervernetzung, eine Amin-reaktive Quervernetzung, eine Quervernetzung zwischen nicht-klassischen Aminosäuren, die in das Peptid, das Protein oder den Proteinkomplex eingeführt worden sind, eine Homo-Quervernetzung, eine Hetero-Quervernetzung oder eine fotoreaktive Quervernetzung aufweist.

9. Verfahren nach irgendeinem der vorstehenden Ansprüche, wobei zumindest eine Quervernetzung eine Dityrosin-Quervernetzung zwischen zwei Aminosäuren in dem Peptid, dem Protein oder dem Proteinkomplex aufweist.

## Revendications

1. Procédé de réduction de l'immunogénicité d'un peptide, d'une protéine ou d'un complexe protéique, comprenant :
(1) l'identification ou la sélection d'au moins un épitope immunogène du peptide, de la protéine ou du complexe protéique à réticuler ; et
(2) l'introduction d'au moins une réticulation entre deux acides aminés se trouvant dans le peptide, la protéine ou le complexe protéique, procédé selon lequel la réticulation est stable ou irréversible *in vivo* et selon lequel la réticulation réduit ou inhibe l'immunogénicité du au moins un épitope par rapport à l'immunogénicité du au moins un épitope se trouvant dans un peptide identique, une protéine identique ou un complexe protéique identique qui n'est pas réticulé de cette manière, et le peptide réticulé, la protéine réticulée ou le complexe protéique réticulé est modifié de manière à ce que les fragments protéolytiques produits par traitement de l'antigène démontrent une moindre capacité de chargement de peptide ou de présentation de peptide sous l'action de HLA-DM ou soient sensibilisés au montage de peptide par HLA-DM ; ou toute combinaison de ceux-ci, en comparaison avec un peptide identique, une protéine identique ou un complexe protéique identique qui n'est pas réticulé de cette manière lors de son administration à un individu.

2. Procédé selon la revendication 1, dans lequel l'épitope est choisi parmi tout peptide au sein de la séquence peptidique ou protéique ayant la capacité de se lier au sillon de la molécule de CMH à des fins de présentation au système immunitaire.

3. Procédé selon l'une quelconque des revendications précédentes, dans lequel un repliement dans le peptide, dans la protéine ou dans le complexe protéique est stabilisé par la réticulation.

4. Procédé selon l'une quelconque des revendications précédentes, comprenant en outre l'introduction d'au moins une autre modification dans le peptide, la protéine ou le complexe protéique, choisie dans le groupe constitué par une mutation ponctuelle, la délétion d'un ou de plusieurs acides aminés, l'insertion d'un ou de plusieurs acides aminés ou une dérivation comprenant une pégylation, une glycosylation, une acétylation, une amidation et une formylation ; ou leurs combinaisons ; dans lequel la au moins une autre modification réduit la dégradation protéolytique induite par les endopeptidases, le chargement de peptide ou la présentation de peptide sous l'action de HLA-DM, la dégradation protéolytique induite par les exopeptidases ou leurs combinaisons ; sensibilise au montage de peptide par HLA-DM ; réduit la formation des complexes de lymphocytes T ou l'activation des lymphocytes T en réponse au peptide, à la protéine ou au complexe protéique, ou les deux ; réduit l'activation des lymphocytes T en réponse au peptide, à la protéine ou au complexe protéique ; réduit l'immunogénicité du peptide, de la protéine ou du complexe protéique ; ou toute combinaison de ceux-ci, en comparaison avec un peptide identique, une protéine identique ou un complexe protéique identique qui n'est pas modifié de cette manière.

5. Procédé selon l'une quelconque des revendications 1, 2 ou 3, dans lequel le peptide réticulé, la protéine réticulée ou le complexe protéique réticulé conserve au moins une fonction du peptide, de la protéine ou du complexe protéique en l'absence de la au moins une réticulation.

6. Procédé selon la revendication 4, dans lequel le peptide modifié, la protéine modifiée ou le complexe protéique modifié conserve au moins une fonction du peptide, de la protéine ou du complexe protéique en l'absence de la au moins une réticulation et de la au moins une autre modification.

7. Procédé selon l'une quelconque des revendications précédentes, dans lequel le peptide, la protéine ou le complexe protéique comprend un produit thérapeutique, un produit de diagnostic, une enzyme, une hormone, un récepteur, un facteur de croissance ou un anticorps ou l'un de ses fragments.

8. Procédé selon l'une quelconque des revendications précédentes, dans lequel au moins une réticulation est choisie dans le groupe comprenant une réticulation oxydative, une réticulation par réaction avec un thiol, une réticulation par réaction avec une amine, une réticulation entre des acides aminés non classiques incorporés dans le peptide, la protéine ou le complexe protéique, une homoréticulation, une hétéroréticulation et une réticulation par photoréaction.

9. Procédé selon l'une quelconque des revendications précédentes, dans lequel au moins une réticulation comprend une réticulation di-tyrosine entre deux acides aminés se trouvant dans le peptide, la protéine ou le complexe protéique.
